# EUROPEAN PATENT APPLICATION

(11) **EP 2 727 916 A1**
(43) Date of publication of application: **07.05.2014**
(21) Application number: 12190483.3
(22) Date of filing: 30.10.2012
(51) Int. Cl.: C07D 401/12, A61K 31/4545, A61P 25/28

(54) **Neuroprotective multi-target directed drugs**

(71) Applicant: Universitat Autònoma de Barcelona, 08193 Bellaterra (Cerdanyola del Vallès) (ES); Consejo Superior de Investigaciones Cientificas (CSIC), 28006 Madrid (ES); National University Corporation Okayama University, Kita-ku Okayama-shi Okayama 700-8530 (JP); Universidad de Alcalá, 28801 Alcala de Henares (Madrid) (ES)
(72) Inventor: Esteban, Gerard, 08013 Barcelona (ES); Unzeta, Mercedes, 08193 Cerdanyola del Vallès (ES); Inokuchi, Tsutomu, Okayama, 700-8530 (JP); Marco-Contelles, José Luis, 28006 Madrid (ES); Samadi, Abdelouahid, 28006 Madrid (ES); Iriepa, Isabel, 28801 Alcalá de Henares (ES); Ojima, Masaki, Okayama, 700-8530 (JP); Li, Wang, Okayama, 700-8530 (JP)
(74) Representative: Vallvé Sánchez, Xavier

(57) **Abstract**

A new family of multitarget molecules able to interact with acetylcholinesterase (AChE) and butyrylcholinesterase (BuChE), as well as with monoamino oxidase (MAO) A and B, has been synthesized. Novel compounds have been designed using a conjunctive approach that combines the benzylpiperidine moiety of the AChE inhibitor donepezil, connected through an oligomethylene linker, to a central nitrogen atom substituted with the propargyl moiety responsible for the MAO inhibition, and a 8-hydroxy-5-methylaminoquinoline functional group, the biometal pro-chelator motif. Overall, the results suggest that the new compounds are promising multitarget drug candidates with potencial impact for AD therapy.

## Description

The present invention relates to multi-target directed ligands useful in the prevention and/or treatment of diseases, disorders and conditions mediated by biometal chelation, brain MAO inhibition and/or cholinesterase inhibition.

### BACKGROUND ART

Alzheimer's disease (AD) is an age-related neurodegenerative process characterized by a progressive memory loss, and other cognitive impairments. Although its etiology is not known, amyloid-β (Aβ) deposits, τ-protein aggregation, oxidative stress and deficits of acetylcholine (ACh) are thought to play significant roles in the pathophysiology of AD. The cholinergic theory suggests that the loss of cholinergic neurons in AD results in low levels of ACh in specific brain regions that mediate learning and memory functions. Consequently, AD patients have been treated with acetylcholinesterase inhibitors (AChEI) tacrine, rivastigmine, donepezil, and galanthamine, but with limited success. This is possibly due to the multifactorial nature of AD, a fact that has prompted the search for new Multi-Target-Directed Ligands (MTDL), based on the "one molecule, multiple target" paradigm. Thus, in this context, for instance, multipotent molecules able to simultaneously bind both cholinesterases and monoamine oxidases have been investigated.

Monoamine oxidase (MAO; EC 1.4.3.4) is an important enzyme to be considered for the treatment of AD, as catalyzes the oxidative deamination of a variety of biogenic and xenobiotic amines, with the concomitant production of hydrogen peroxide, a key intermediate via Fenton reaction in the production of toxic radical oxygenated species, clearly implicated in the progress of AD. MAO is a FAD-containing enzyme bound to mitochondrial outer membrane of neuronal, glia and other cells. MAO exists as two isozymes, MAO-A and MAO-B, showing different substrate specificity, sensitivity to inhibitors, and aminoacid sequences. MAO-A preferentially oxidizes norepinephrine and serotonine, and is selectively inhibited by chlorgyline, while MAO-B preferentially deaminates β-phenylethylamine and is irreversibly inhibited by I-deprenyl. X-ray crystal structures of rat MAO-A and human MAO-B have been reported. In this context, and using the MTDL approach, a very successful approach came from the combination of the carbamate moiety of rivastigmine with the indolamine moiety present in rasagiline, a well-known MAO-B inhibitor, leading to ladostigil. The potential therapeutic effect of this compound, which has reached clinical trials, is also supported by recent findings showing the ability of propargylamine-containing compounds to modulate cleavage of Aβ protein precursor. New hybrid compounds targeting MAO and AChE and site-activated chelators targeting MAO have also been recently described.

Several studies have demonstarted the role of biometals dyhosmeostasis in most neurodegenerative disorders. The etiology of AD is characterized by the-abnormally elevated concentrations of biometals such as copper or iron by spectroscopy studies which actively promote the aggregation of β-amyloid peptides leading to the formation of senile plaques, the production of oxygen reactive species (ROS) and oxidative stress.

Modulation of such biometal levels in the brain have been proposed as a potential therapeutic strategy for the treatment of AD.

Numerous studies, including *in vivo, in vitro* and animal models have shown a linkage between MAO, AChE and biometal chelation with neurodegenerative diseases and disorders, such as Parkinson's disease (PD) and Alzheimer's disease (AD) among others.

New multi-target ligands able to simultaneously bind both cholinesterases and monoamine oxidases and with biometal quelation ability are currently object of study. Most of the MTDL under study comprise two or more functional moieties which imparts biometal chelation functions, MAO inhibition functions and AChE inhibition function. Thus, in Youdim M.B.H. et al., "Bifunctional drug derivatives of MAO-B inhibitor rasagiline and iron chelator VK-28 as a more effective approach to treatment of brain ageing and ageing neurodegenerative diseases", Mechanisms of Ageing and Development 2005, 126, 317-326, it is stated that similar to treatment of cancer, AIDs, cardiovascular diseases and neuropsychiatric diseases where the use of multi-drug therapy is now a common place, neuroprotection in neurodegenerative diseases may require either a similar approach or the use of bi or trifunctional drugs. Neuroprotective activity of bifunctional cholinesterase-MAO inhibitors and MAO-iron chelation derivatives is discussed.

In the international patent application WO2004/41151 (Yeda Res. Dev. Co.) compounds M30 and HLA20 and analogues have been described:

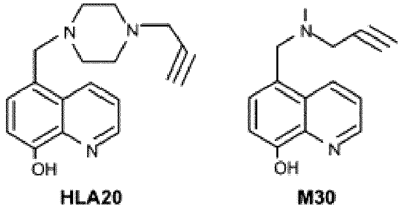

These compounds are MAO inhibitors and iron chelators. These compounds are claimed to be useful for the treatment of AD among other diseases, disorders or conditions related to iron overload and oxidative stress.

WO2010/86860 (Yeda Res. Dev. Co.) discloses multifunctional compounds incorporating (i) a moiety that imparts an iron chelator function; (ii) a moiety that imparts a neuroprotective function; (iii) a moiety that imparts combined antiapoptotic, neuroprotective and/or neurorestorative functions; (iv) a moiety that imparts brain MAO inhibition; (v) a moiety that imparts ChE inhibitory function; and (vi) a moiety that imparts NMDAR inhibition. Among the diseases, disorders and conditions to be treated with the claimed compounds, Alzheimer's disease is specifically mentioned.

It would be very desirable to provide compounds having a multi-targeted activity, that exhibit also good transport properties through cell membranes including the blood brain barrier as drug candicates for clinical development.

### SUMMARY OF THE INVENTION

The present inventors have been successful in finding a novel series of compounds which are multipotent hybrids having good multi-targed activity, being able to interact in two key enzymatic systems implicated in neurodegenerative and cerebrovascular disorders, diseases and/or conditions, and showing certain properties that make them particularly useful in formulating medicaments in the treatment of such disorders, diseases and/or conditions. The new compounds according to the present invention are multi-target directed ligands which simultaneously bind amino oxidases (MAO) as well as cholinesterases (AChE, BuChE).

Furthermore, the compounds of the invention of formula (I) are specific biometal chelators that are suitable to bind biometals within the cells. The compounds of the invention have good transport properties and cross cell membranes thus chelating the biometal (particularly iron, copper and zinc) in excess within the cells. It is expected that their complexes with biometals will leave the cells freely and will be rapidly excreted. It is further expected that the compounds of the present invention will be able to cross the blood brain barrier (BBB) and thus will be suitable candidates for the treatment of neurodegenerative diseases, disorders and conditions.

Due to its biometal chelation properties, the compounds of the present invention are neuroprotective chelators with the capacities of modulating β-amyloid protein precursor (APP) regulation, Aβ reduction, and inhibiting Aβ aggregation induced by biometals Fe, Cu and Zn, with activities against oxidative stress, metal toxicity, and biometal dyshomeostasis in the brain.

Thus, in a first aspect the present invention provides a compound of formula (I) 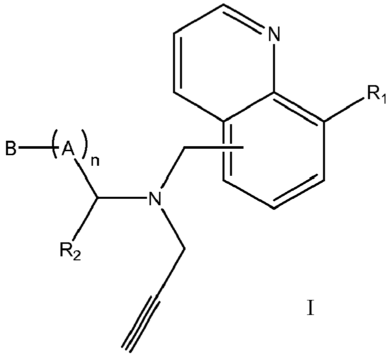 or a salt thereof, or any of its stereoisomeric forms or a mixture thereof, wherein
R₁ and R₂, same or different, are each independently selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; halo-(C₁-C₆)alkyl; linear or branched (C₂-C₆)alkenyl; F; Cl; Br; I; -NO₂; -NH₂; -CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl, - SON((C₁-C₄)alkyl)₂; -OCONH₂; -OCONH(C₁-C₄)alkyl; -OCON((C₁-C₄)alkyl)₂; - C=N(C₁-C₄)alkyl; -NHC(O)(C₁-C₄)alkyl; -N(C₁-C₄)alkylC(O)(C₁-C₄)alkyl; - N(C₁-C₄)alkylC(O)H; -N=CH₂; -N=CH(C₁-C₄)alkyl; -N=C((C₁-C₄)alkyl)₂; phenylcarbonyl; phenylcarbonyl, the phenyl moiety being substituted by at least a radical selected from the group consisting of halo(C₁-C₆)-alkyl, OH, halogen, (C₁-C₆)-alkyl, (C₁-C₆)-alkoxy, and CN; (C₁-C₃)-alkylphenylcarbonyl; (C₁-C₃)-alkylphenylcarbonyl being the phenyl moiety substituted by at least a radical selected from the group consisting of halo(C₁-C₆)-alkyl, OH, halogen, (C₁-C₆)-alkyl, (C₁-C₆)-alkoxy, and CN; phenyl; phenyl substituted by at least a radical selected from the group consisting of halo(C₁-C₆)-alkyl, OH, halogen, (C₁-C₆)-alkyl, (C₁-C₆)-alkoxy, and CN; (C₁-C₃)-alkylphenyl; (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN;
n is an integer from 0 to 1;
A is selected from the group consisting of
(i) -(CH₂)ₙ₁-X-(CH₂)ₙ₂- wherein n1 and n2 are an integer each independently selected from 0 to 6;
(ii) -X-(CH₂)ₙ₃-Y- wherein n3 is an integer from 1 to 6;
(iii) a bivalent radical selected from the group consisting of 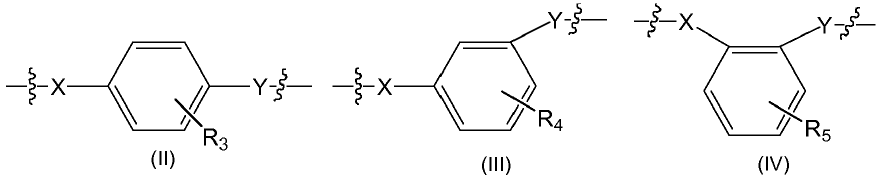 wherein R₃, R₄ and R₅ are selected from H, OH, halogen, CN, (C₁-C₄)alkyl, hydroxy-(C₁-C₄)alkyl; halo(C₁-C₆)-alkyl, and (C₁-C₆)alkoxy;
   wherein X and Y are each independently selected from O, N and CH₂;
   and wherein the wavy line indicates the position through which the A moiety binds, being the X atom bound to the B moiety and the Y atom bound to the backbone of the compound of formula (I);
   B is selected from the group consisting of 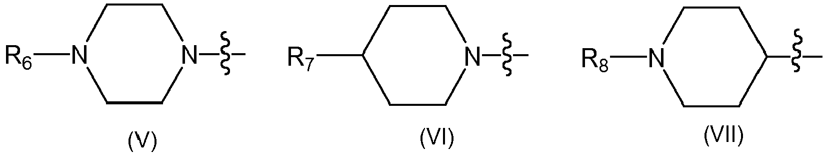 wherein the wavy line indicates the position through which the B moiety binds to the X atom of the A moiety, and wherein R₆, R₇ and R₈ are selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; halo-(C₁-C₆)alkyl; linear or branched (C₂-C₆)alkenyl; F; Cl; Br; I; -NO₂; -NH₂; -CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C ₄)alkyl; -SO₂-(C₁-C₄)alkyl; -SO₂NH₂; -SONH(C₁-C₄)alkyl; - SON((C₁-C₄)alkyl)₂; -OCONH₂; -OCONH(C₁-C₄)alkyl; -OCON((C₁-C₄)alkyl)₂; - C=N(C₁-C₄)alkyl; -NHC(O)(C₁-C₄)alkyl; -N(C₁-C₄)alkylC(O)(C₁-C₄)alkyl; - N(C₁-C₄)alkylC(O)H; -N=CH₂; -N=CH(C₁-C₄)alkyl; -N=C((C₁-C₄)alkyl)₂; phenylcarbonyl; phenylcarbonyl, the phenyl moiety being substituted by at least a radical selected from the group consisting of halo(C₁-C₆)-alkyl, OH, halogen, (C₁-C₆)alkyl, (C₁-C₆)alkoxy, and CN; (C₁-C₃)-alkylphenylcarbonyl; (C₁-C₃)-alkylphenylcarbonyl being the phenyl moiety substituted by at least a radical selected from the group consisting of halo(C₁-C₆)-alkyl, OH, halogen, (C₁-C₆)alkyl, (C₁-C₆)alkoxy, and CN; phenyl; phenyl substituted by at least a radical selected from the group consisting of halo(C₁-C₆)-alkyl, OH, halogen, (C₁-C₆)alkyl, (C₁-C₆)alkoxy, and CN; (C₁-C₃)-alkylphenyl; (C₁-C₃)-alkylphenyl, being the phenyl moiety substituted by at least a radical selected from the group consisting of halo(C₁-C₆)-alkyl, OH, halogen, (C₁-C₆)alkyl, (C₁-C₆)alkoxy, and CN; and a C-radical of one of the known carbocyclic or heterocyclic ring systems with 1-4 rings, wherein each one of the rings forming said ring system:
   has 5-7 members, each member independently selected from C, N, O, S, CH, CH₂, NH,
   is saturated, partially unsaturated or aromatic, and
   is isolated, partially or totally fused;
   being each ring forming part of the ring system optionally substituted by at least one radical selected from the group consisting of H;
   OH; -CH₂-NH-CH₂CCH; -NH-
   CH₂CCH; -CH₂-N(C₁-C₄)alkylCH₂CCH; -N(C₁-C₄)alkylCH₂CCH;linear or branched (C₁-C₆)alkyl; hydroxy-(C₁-C₆)alkyl; halo-(C₁-C₆)alkyl; linear or branched (C₂-C₆)alkenyl; F; Cl; Br; I; -NO₂; -NH₂; -CN; -O(C₁-C₄)alkyl; (C₁-C₄)alkyl-O-(C₁-C₄)alkyl; -
   C(O)H; -C(O)(C₁-C₄)alkyl; -COOH; -COO(C₁-C₄)alkyl; -OC(O)H; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C ₄)alkyl; -SO₂-(C₁-C₄)alkyl; -SO₂NH₂; -SONH(C₁-C₄)alkyl; - SON((C₁-C₄)alkyl)₂; -OCONH₂; -OCONH(C₁-C₄)alkyl; -OCON((C₁-C₄)alkyl)₂; - C=N(C₁-C₄)alkyl; -NHC(O)(C₁-C₄)alkyl; -N(C₁-C₄)alkylC(O)(C₁-C₄)alkyl; - N(C₁-C₄)alkylC(O)H; -N=CH₂; -N=CH(C₁-C₄)alkyl; -N=C((C₁-C₄)alkyl)₂;
   with the proviso that wherein the A moiety is -X-(CH₂)_{n"}-Y-, or a radical of formula (II), (III) or (IV) and X is N or O, then B is a radical of formula (VII).

Another aspect of the invention relates to a pharmaceutical composition which comprises at least one compound of formula (I) or a pharmaceutically acceptable salt thereof and one or more pharmaceutically acceptable excipients, carriers or mixtures thereof.

A third aspect of the invention provides a compound according to the first aspect of the invention for use as an inhibtor of amino oxidases (MAO), an inhibitor of semicarbazido-sensitive amine oxidase (SSAO), an inhibitor of cholinesterases (AChE, BuChE) and/or a biometal chelator, specifically for use as a medicament.

A fourth aspect of the invention is the use of a compound of formula (I), for the manufacture of a medicament for the treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder. This aspect of the invention can also be formulated as compounds of formula (I) as defined in the first aspect of the invention for use in the treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder.

Alternatively, this aspect may be formulated as a method for treating a disorder, disease and/or condition mediated by inhibition of amino oxidases (MAO), inhibition of cholinesterases (AChE, BuChE) and/or biometal chelation in a mammal in need thereof, especially a human, such disorder, disease and/or condition being preferably a neurodegenerative disorder, which comprises administering to the patient a compound of formula (I) or a pharmaceutically acceptable salt thereof together with pharmaceutical excipients, carriers or mixtures thereof.

An additional aspect of the invention provides the use of a compound of formula (I) in the preparation of a medicament for the treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder, wherein said treatment comprises administering to a subject simultaneously, sequentially or separately at least one compound of formula (I) as defined above and one or more active agents useful in conventional treatment of such a disorder, disease and/or condition.

Alternatively, this aspect can be formulated as to provide a compound of formula (I) for use in a treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder, wherein said treatment comprises administering to a subject simultaneously, sequentially or separately at least one compound of formula (I) as defined above and one or more active agents useful in conventional treatment of such a disorder, disease and/or condition.

Further provided is a method of treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder, comprising administering simultaneously, sequentially or separately, to a mammal, including a human, suffering such a disorder, disease and/or condition a therapeutically effective quantity of at least one compound of formula (I) as defined above and one or more active agents useful in conventional treatment of such a disorder, disease and/or condition

Another aspect of the invention provides the use of a combined preparation comprising at least one compound of formula (I) as defined above and one or more active agents useful in conventional treatment of such a disorder, disease and/or condition, in the manufacture of a medicament for simultaneous, sequential or separated use in the treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder. Alternatively, this aspect can be formulated as to provide a combined preparation comprising at least one compound of formula (I) as defined above and one or more active agents useful in conventional treatment of such a disorder, disease and/or condition, for simultaneous, sequential or separated use in the treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder.

Further provided is a method of treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder, comprising administering simultaneously, sequentially or separately, to a mammal, including a human, suffering such a disorder, disease and/or condition a therapeutically effective quantity of a combined preparation comprising at least one compound of formula (I) as defined above and one or more active agents useful in conventional treatment of such a disorder, disease and/or condition.

Another aspect relates to the use of a compound of formula (I) in a combined therapy which comprises the use of at least one compound of formula (I) as defined above and one or more active agents useful in conventional treatment a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder.

Alternatively, this aspect can be formulated as to provide a compound of formula (I) for use in a treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder, in a combination therapy which comprises the use of a compound of formula (I) and one or more active agents useful in conventional treatment of such a disorder, disease and/or condition.

Further provided is a method of treatment of a disorder, disease and/or condition mediated by inhibition of amino oxidases (MAO), inhibition of cholinesterases (AChE, BuChE) and/or biometal chelation, such disorder, disease and/or condition being preferably a neurodegenerative disorder, comprising a combined therapy of a compound of formula (I) and one or more active agents useful in conventional treatment of such a disorder, disease and/or condition.

Yet another aspect of the present invention relates to a process for the preparation of a compound of formula (I), which comprises reductive amination of the aldehyde of formula (IX) with propargylamine, followed by N-alkylation with 5-chloromethyl-quinolin-8-yl substituted compound of formula (X)

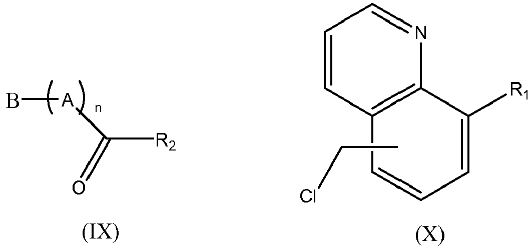

Alternatively, compounds of formula (I), can be prepared starting from the 5-(chloromethyl)quinolin-8-yl derivative of formula (X), by reaction with propargylamine to give the 5-((prop-2-yn-1-ylamino)methyl)quinolin-8-yl derivative of formula (XI), followed by reductive amination with aldehydes of formula (IX)

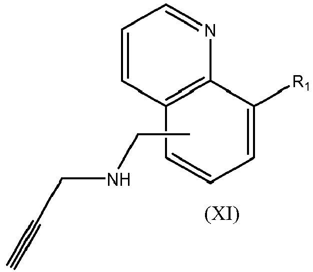

Alternatively, compounds of formula (I), can be prepared by reaction of compounds of formula (XII) with propargylamine to provide intermediates of type (XIII), and reaction with chlorides of general structure (XIV).

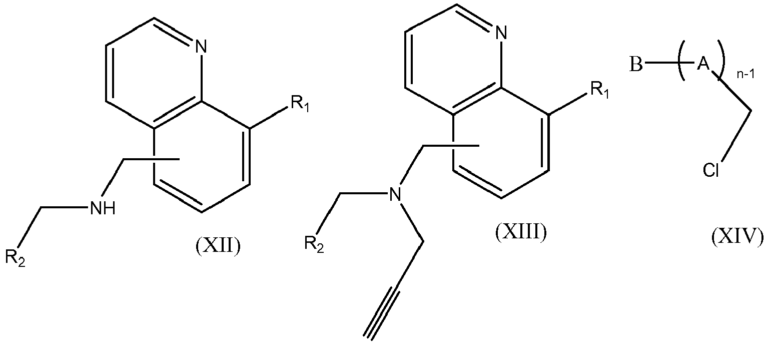

### BRIEF DESCRIPTION OF THE DRAWINGS

FIG. 1 Steady-state inhibition of EeAChE hydrolysis of acetylthiocholine (ATCh) by compound 6. Lineweaver-Burk reciprocal plots of initial velocity and substrate concentrations (0.1-1 mM) are presented. Lines were derived from a weighted least-squares analysis of data.
FIG. 2 Steady-state inhibition of rat liver MAO-A by compound 6.
   Lineweaver-Burk reciprocal plots of initial velocity and substrate concentrations (0.1-1 mM) are presented. Lines were derived from a weighted least-squares analysis of data.
FIG. 3 Steady-state inhibition of rat liver MAO-B by compound 6.
   Lineweaver-Burk reciprocal plots of initial velocity and substrate concentrations (0.1-1 mM) are presented. Lines were derived from a weighted least-squares analysis of data.

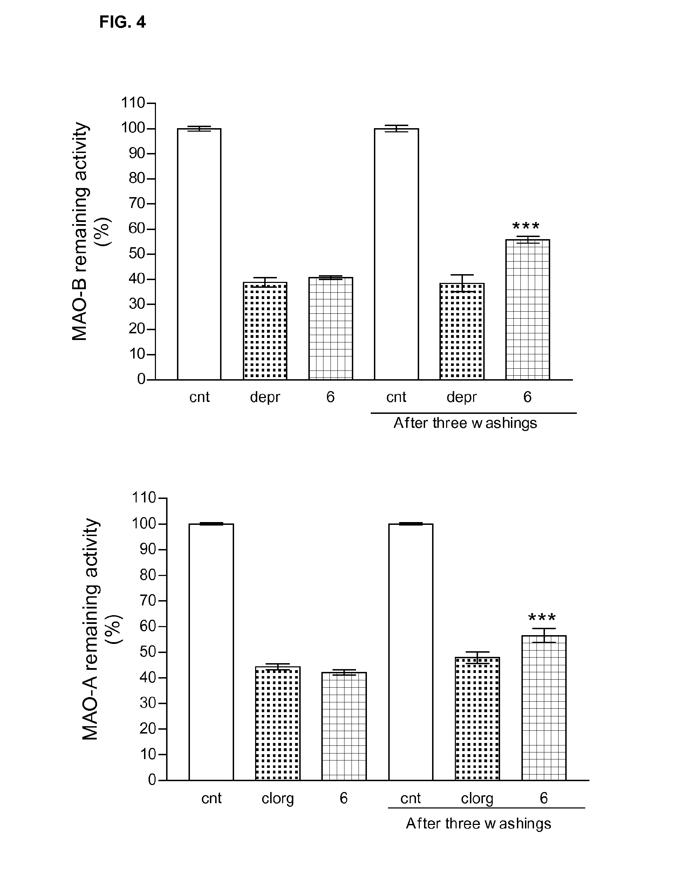

FIG. 4 Reversibility studies of MAO-A and MAO-B inhibition by compounds 6, clorgyline and I-deprenyl. MAO-A and MAO-B were inhibited at 10 and 20 µM respectively with 6 and 20 and 50 nM with I-deprenyl and clorgyline respectively, for 30 min in all cases. Three consecutive centrifuges were performed with buffer. Both MAO-A and MAO-B activities were significantly reverted with 6 (recovering 56.5 ± 9.3 % in A and 55.8 ± 4.0 % in B) and, as expected, MAO-A and B activities remained unaltered when clorgyline and I-deprenyl were used. Data are the mean ± SEM of four or five independent experiments in triplicate. ***P <0.01 significantly compared to untreated controls.
FIG. 5 Time-dependence inhibition was performed at different pre-incubation times (0-360 min) of MAO with compound 6. Both MAO-A and MAO-B activities proved to be time-dependent when inhibited by compound **6.** Data are the mean ± SEM of three independent experiments in triplicate.
FIG. 6 Time-dependence inhibition was performed at different times of pre-incubation (0-240 min) of MAO with compound **6.** Both MAO-A and MAO-B proved to be time-dependent. Data are the mean ± SEM of three independent experiments in triplicate.
FIG. 7 Spectrophotometric detection of Fe(III) and Cu(II) complexation with compound **6:**
   **a)** (a) UV-vis spectrum of compound **6** (10 µM); (b) Compound **6** (10 µM) + Fe₂(SO₄)₃ (6.25 µM); (c) Compound **6** + Fe₂(SO₄)₃ (12. 5 µM). All solution were made using Tris buffer pH7.4. Spectra were obtained after a Short-time incubation
   **b)** (a) UV-vis spectrum of compound **6** (10 µM); (b) Compound **6** (10 µM) + Fe₂(SO₄)₃ (6.25 µM); (c) Compound **6** + Fe₂(SO₄)₃ (12. 5 µM). All solution were made using Tris buffer pH7.4. Spectra were obtained after an overnight incubation
   **c)** (a) UV-vis spectrum of compound **6** (10 µM); (b) Compound **6** (10 µM) + Cu(SO₄) (6.25 µM); (c) Compound **6** + Cu(SO₄) (12. 5 µM). All solution were made using Tris buffer pH7.4. Spectra were obtained after a Short-time incubation

### DETAILED DESCRIPTION OF THE INVENTION

As it has been stated above, in a first aspect the present invention provides compounds of formula (I), or a salt thereof, or any of its stereoisomeric forms or a mixture thereof.

According to a preferred embodiment of the present invention, the compounds of formula (I) are those wherein B is a radical of formula (VII), and wherein R₈ is a radical as defined above.

In accordance with a more preferred embodiment, the compounds are those wherein R₈ is selected from the group consisting of H; OH; -CH₂-NH-CH₂CCH; -NH-CH₂CCH; -CH₂-N(C₁-C₄)alkylCH₂CCH; - N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)NHCH₃; -C(O)N(CH₃)₂; -NH₂; -NHCH₃; -N(CH₃)₂; - CH₂NH₂; -CH₂CH₂NH₂; -CH₂NHCH₃; -CH₂N(CH₃)₂; -CH₂CH₂N(CH₃)₂; - CH₂N(CH₂CH₃)₂; -CH₂CH₂N(CH₂CH₃)₂; -SH; -SCH₃; -SCH₂CH₃; -SO-CH₃; - SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH3)₂; -C=NH; -C=NCH₃; - C=NCH₂CH₃; -NCH₃C(O)CH₃; -NCH₃C(O)CH₂CH₃; -N=CH₂; -N=C(CH₃)₂; - N=C(CH₂CH₃)₂; phenylcarbonyl; phenylcarbonyl, the phenyl moiety being substituted by at least a radical selected from the group consisting of CF₃, F, Cl, Br, I, CN, OH, -CH₃, -CH₂CH₃, -OCH₃, and -OCH₂CH₃; - (C₁-C₃)-alkylphenylcarbonyl, (C₁-C₃)-alkylphenylcarbonyl being the phenyl moiety substituted by at least a radical selected from the group consisting of CF₃, F, Cl, Br, I, CN, OH, -CH₃, -CH₂CH₃, -OCH₃, and -OCH₂CH₃; phenyl; phenyl substituted by at least a radical selected from the group consisting of CF₃, F, Cl, Br, I, CN, OH, -CH₃, -CH₂CH₃, -OCH₃, and -OCH₂CH₃; (C₁-C₃)-alkylphenyl; (C₁-C₃)-alkylphenyl, being the phenyl moiety substituted by at least a radical selected from the group consisting of CF₃, F, Cl, Br, I, CN, OH, -CH₃, -CH₂CH₃, -OCH₃, and -OCH₂CH₃.

In accordance with another preferred embodiment, R₈ is a C-radical of one of the known carbocyclic or heterocyclic ring systems with 1-2 rings, wherein each one of the rings forming said ring system:
has 5-6 members, each member independently selected from C, N, O, S, CH, CH₂, NH,
is saturated, partially unsaturated or aromatic,
being each ring forming part of the ring system optionally substituted by at least one radical selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; -C(O)H; -C(O)(C₁-C₄)alkyl; -COOH; -COO(C₁-C₄)alkyl; -OC(O)H; -OC(O)(C₁-C₄)alkyl; -C(O)NHCH₃; -C(O)N(CH₃)₂; -NH₂; - NHCH₃; -N(CH₃)₂; -CH₂NH₂; -CH₂CH₂NH₂; -CH₂NHCH₃; -CH₂N(CH₃)₂; - CH₂CH₂N(CH₃)₂; -CH₂N(CH₂CH₃)₂; -CH₂CH₂N(CH₂CH₃)₂; -SH; -SCH₃; - SCH₂CH₃; -SO-CH₃; -SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; -C=NH; - C=NCH₃; -C=NCH₂CH₃; -NCH₃C(O)CH₃; -NCH₃C(O)CH₂CH₃; -N=CH₂; - N=C(CH₃)₂; -N=C(CH₂CH₃)₂.

Being particularly preferred those compounds wherein R₈ is selected from pyrrolyl, furanyl, thiophenyl, pyrazolyl, imidazolyl, triazolyl, tetrazolyl, oxazolyl, isoxazolyl, thiazolyl, isothiazolyl, dithiolanyl, pyridinyl, pyranyl, pyridazinyl, pyrimidinyl, pyrazinyl, piperazinyl, benzofuranyl, isobenzofuranyl, indolyl, indenyl, dihydroindenyl, naphthalenyl, isoindolyl, indolizinyl, indolinyl, isoindolinyl, adeninyl, guaninyl, benzimidazolyl, indazolyl, benzoxazolyl, benzisoxazolyl, benzodioxolyl, benzofurazanyl, benzotriazolyl, benzothiofuranyl, benzothiazolyl, benzothiadiazolyl, chromenyl, isochromenyl, chromanyl, isochromanyl, benzodioxanyl, quinolinyl, and isoquinolinyl; being each ring forming part of the ring system optionally substituted by at least one radical selected from the group consisting of H; OH; -CH₂-NH-CH₂CCH; -NH-CH₂CCH; -CH₂-N(C₁-C₄)alkylCH₂CCH; - N(C₁-C₄)alkylCH₂CCH;linear or branched (C₁-C₆)alkyl; hydroxy-(C₁-C₄)alkyl; CF₃; CN; -O(C₁-C₄)alkyl; (C₁-C₄)alkyl-O-(C₁-C₄)alkyl; -C(O)H; -C(O)(C₁-C₄)alkyl; -COOH; -COO(C₁-C₄)alkyl; - OC(O)H; -OC(O)(C₁-C₄)alkyl; -C(O)NHCH₃; -C(O)N(CH₃)₂; -NH₂; -NHCH₃; - N(CH₃)₂; -CH₂NH₂; -CH₂CH₂NH₂; -CH₂NHCH₃; - CH₂N(CH₃)₂; -CH₂CH₂N(CH₃)₂; -CH₂N(CH₂CH₃)₂; -CH₂CH₂N(CH₂CH₃)₂; -SH; -SCH₃; -SCH₂CH₃; -SO-CH₃; -SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; - C=NH; -C=NCH₃; -C=NCH₂CH₃; -NCH₃C(O)CH₃; -NCH₃C(O)CH₂CH₃; - N=CH₂; -N=C(CH₃)₂; -N=C(CH₂CH₃)₂.

A particular preferred group of compounds of the present invention are those wherein R₈ is a quinolinyl of formula (VIII) 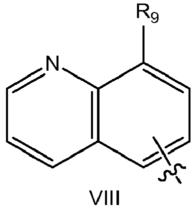 wherein the wavy line indicates the position through the quinolinyl radical of formula (VIII) binds to nitrogen atom of the radical of formula (VII); and wherein the R₉ radical is selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; -C(O)H; -C(O)(C₁-C₄)alkyl; -COOH; -COO(C₁-C₄) alkyl; -OC(O)H; -OC(O)(C₁-C₄)alkyl; -C(O)NHCH₃; -C(O)N(CH₃)₂; -NH₂; - NHCH₃; -N(CH₃)₂; -CH₂NH₂; -CH₂CH₂NH₂; -CH₂NHCH₃; - CH₂N(CH₃)₂; -CH₂CH₂N(CH₃)₂; -CH₂N(CH₂CH₃)₂; -CH₂CH₂N(CH₂CH₃)₂; -SH; -SCH₃; -SCH₂CH₃; -SO-CH₃; -SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; -

C=NH; -C=NCH₃; -C=NCH₂CH₃; -NCH₃C(O)CH₃; -NCH₃C(O)CH₂CH₃; - N=CH₂; -N=C(CH₃)₂; -N=C(CH₂CH₃)₂.

According to another embodiment of the present invention, the compounds of formula (I) are those wherein n is an integer from 0 to 1, and the B-A moiety is selected from the group consisting of B-(CH₂)ₙ₁-O-(CH₂)ₙ₂-; B-(CH₂)ₙ₁-NH-(CH2)n2-; B-(CH₂)ₙ₁-CH₂-; B-(CH₂)ₙ₁-O-; B-(CH₂)ₙ₁-NH-; B-O-(CH₂)ₙ₂-; B-NH-(CH₂)ₙ₂-; B-; B-O-; B-NH-; B-O-(CH₂)ₙ₃-O-; B-O-(CH₂)ₙ₃-NH-; B-NH-(CH₂)ₙ₃-NH-; and B-NH-(CH₂)ₙ₃-O-;
being B as defined above; and n1, n2 and n3 are an integer each independently selected from 1 to 4.

In accordance with a particularly preferred embodiment, the compounds are those wherein n is an integer from 0 to 1, and the B-A moiety is selected from the group consisting of B-(CH₂)ₙ₁-O-(CH₂)ₙ₂-; B-(CH₂)ₙ₁-NH-(CH₂)ₙ₂-; B-(CH₂)ₙ₁-CH₂-; B-(CH₂)ₙ₁-O-; B-(CH₂)ₙ₁-NH-; B-O-(CH₂)ₙ₂-; B-NH-(CH₂)ₙ₂-; B-; B-O-; B-NH-; B-O-(CH₂)ₙ₃-O-; B-O-(CH₂)ₙ₃-NH-; B-NH-(CH₂)ₙ₃-NH-; and B-NH-(CH₂)ₙ₃-O-;
being n1, n2 and n3 are an integer each independently selected from 1 to 4; and B is a radical of formula (VII), being R₈ selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)NHCH₃; -C(O)N(CH₃)₂; -NH₂; -NHCH₃; -N(CH₃)₂; - CH₂NH₂; -CH₂CH₂NH₂; -CH₂NHCH₃; -CH₂N(CH₃)₂; -CH₂CH₂N(CH₃)₂; - CH₂N(CH₂CH₃)₂; -CH₂CH₂N(CH₂CH₃)₂; -SH; -SCH₃; -SCH₂CH₃; -SO-CH₃; - SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; -C=NH; -C=NCH₃; - C=NCH₂CH₃; -NCH₃C(O)CH₃; -NCH₃C(O)CH₂CH₃; -N=CH₂; -N=C(CH₃)₂; - N=C(CH₂CH₃)₂; phenylcarbonyl; phenylcarbonyl, the phenyl moiety being substituted by at least a radical selected from the group consisting of CF₃, F, Cl, Br, I, CN, OH, -CH₃, -CH₂CH₃, -OCH₃, and -OCH₂CH₃; - (C₁-C₃)-alkylphenylcarbonyl, (C₁-C₃)-alkylphenylcarbonyl being the phenyl moiety substituted by at least a radical selected from the group consisting of CF₃, F, Cl, Br, I, CN, OH, -CH₃, -CH₂CH₃, -OCH₃, and -OCH₂CH₃; phenyl; phenyl substituted by at least a radical selected from the group consisting of CF₃, F, Cl, Br, I, CN, OH, -CH₃, -CH₂CH₃, -OCH₃, and -OCH₂CH₃; (C₁-C₃)-alkylphenyl; (C₁-C₃)-alkylphenyl, being the phenyl moiety substituted by at least a radical selected from the group consisting of CF₃, F, Cl, Br, I, CN, OH, -CH₃, -CH₂CH₃, -OCH₃, and -OCH₂CH₃.

In another preferred embodiment, the compounds of formula (I) are those wherein n is 1, A moiety is selected from a bivalent radical selected from the group consisting of 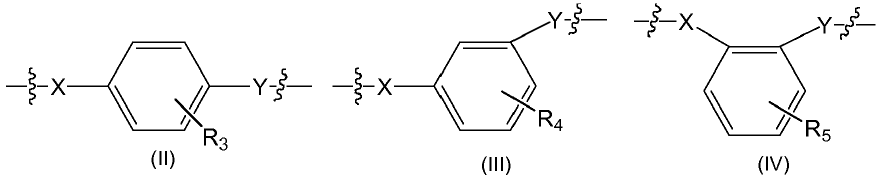 wherein R₃, R₄ and R₅ are selected from H, OH, halogen, CN, (C₁-C₄)alkyl, halo(C₁-C₆)-alkyl, and (C₁-C₆)alkoxy;
wherein X and Y are each independently selected from O, N and CH₂; and wherein the wavy line indicates the position through which the A moiety binds, being the X atom bound to the B moiety and the Y atom bound to the backbone of the compound of formula (I).

In accordance with a particularly preferred embodiment, the compounds are those wherein n is 1, A moiety is selected from a bivalent radical of formula (II), (III) or (IV); wherein R₃, R₄ and R₅ are selected from H, OH, halogen, CN, (C₁-C₄)alkyl, halo(C₁-C₆)-alkyl, and (C₁-C₆)alkoxy; wherein X and Y are each independently selected from O, N and CH₂; and wherein the wavy line indicates the position through which the A moiety binds, being the X atom bound to the B moiety and the Y atom bound to the backbone of the compound of formula I; and wherein B is a radical of formula VII, being R₈ selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)NHCH₃; -C(O)N(CH₃)₂; -NH₂; -NHCH₃; -N(CH₃)₂; - CH₂NH₂; -CH₂CH₂NH₂; -CH₂NHCH₃; -CH₂N(CH₃)₂; -CH₂CH₂N(CH₃)₂; - CH₂N(CH₂CH₃)₂; -CH₂CH₂N(CH₂CH₃)₂; -SH; -SCH₃; -SCH₂CH₃; -SO-CH₃; - SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; -C=NH; -C=NCH₃; - C=NCH₂CH₃; -NCH₃C(O)CH₃; -NCH₃C(O)CH₂CH₃; -N=CH₂; -N=C(CH₃)₂; - N=C(CH₂CH₃)₂; phenylcarbonyl; phenylcarbonyl, the phenyl moiety being substituted by at least a radical selected from the group consisting of CF₃, F, Cl, Br, I, CN, OH, -CH₃, -CH₂CH₃, -OCH₃, and -OCH₂CH₃; - (C₁-C₃)-alkylphenylcarbonyl, (C₁-C₃)-alkylphenylcarbonyl being the phenyl moiety substituted by at least a radical selected from the group consisting of CF₃, F, Cl, Br, I, CN, OH, -CH₃, -CH₂CH₃, -OCH₃, and -OCH₂CH₃; phenyl; phenyl substituted by at least a radical selected from the group consisting of CF₃, F, Cl, Br, I, CN, OH, -CH₃, -CH₂CH₃, -OCH₃, and -OCH₂CH₃; (C₁-C₃)-alkylphenyl; (C₁-C₃)-alkylphenyl, being the phenyl moiety substituted by at least a radical selected from the group consisting of CF₃, F, Cl, Br, I, CN, OH, -CH₃, -CH₂CH₃, -OCH₃, and -OCH₂CH₃.

According to another preferred embodiment of the present invention, the compounds of formula (I) are those wherein R₁ and R₂, same or different, are each independently selected from the group consisting of H; OH; -CH₂-NH-CH₂CCH; -NH-CH₂CCH; -CH₂-N(C₁-C₄)alkylCH₂CCH; - N(C₁-C₄)alkylCH₂CCH; linear or branched (C₁-C₆)alkyl; hydroxy-(C₁-C₆)alkyl; halo-(C₁-C₆)alkyl; -NH₂; -CN; -C(O)(C₁-C₄)alkyl; -COO(C₁-C₄)alkyl; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl; - SON((C₁-C₄)alkyl)₂; -OCONH₂; -OCONH(C₁-C₄)alkyl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN.

Preferably, R₁ is selected from the group consisting of OH; -NH₂; - CN; -C(O)(C₁-C₄)alkyl; -COO(C₁-C₄)alkyl; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl; -SON((C₁-C₄)alkyl)₂; -OCONH₂; -OCONH(C₁-C₄)al kyl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN.

A particularly preferred group of compounds are those of formula (Ia) 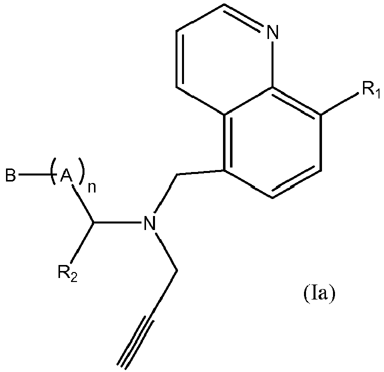 wherein
R₁ is selected from OH; -NH₂; - CN; -C(O)(C₁-C₄)alkyl; -COO(C₁-C₄)alkyl; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl; -SON((C₁-C₄)alkyl)₂; -OCONH₂; -OCONH(C₁-C₄)al kyl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN;
R₂ is selected from H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; halo-(C₁-C₆)alkyl; -NH₂; - CN; -C(O)(C₁-C₄)alkyl; -COO(C₁-C₄)alkyl; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl, - SON((C₁-C₄)alkyl)₂; -OCONH₂; -OCONH(C₁-C₄)alkyl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN;
B is a radical of formula VII, wherein R₈ is selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)NHCH₃; -C(O)N(CH₃)₂; -NH₂; -NHCH₃; -N(CH₃)₂; - CH₂NH₂; -CH₂CH₂NH₂; -CH₂NHCH₃; -CH₂N(CH₃)₂; -CH₂CH₂N(CH₃)₂; - CH₂N(CH₂CH₃)₂; -CH₂CH₂N(CH₂CH₃)₂; -SH; -SCH₃; -SCH₂CH₃; -SO-CH₃; - SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; -C=NH; -C=NCH₃; - C=NCH₂CH₃; -NCH₃C(O)CH₃; -NCH₃C(O)CH₂CH₃; -N=CH₂; -N=C(CH₃)₂; - N=C(CH₂CH₃)₂; phenylcarbonyl; phenylcarbonyl, the phenyl moiety being substituted by at least a radical selected from the group consisting of CF₃, F, Cl, Br, I, CN, OH, -CH₃, -CH₂CH₃, -OCH₃, and -OCH₂CH₃; - (C₁-C₃)-alkylphenylcarbonyl, (C₁-C₃)-alkylphenylcarbonyl being the phenyl moiety substituted by at least a radical selected from the group consisting of CF₃, F, Cl, Br, I, CN, OH, -CH₃, -CH₂CH₃, -OCH₃, and -OCH₂CH₃; phenyl; phenyl substituted by at least a radical selected from the group consisting of CF₃, F, Cl, Br, I, CN, OH, -CH₃, -CH₂CH₃, -OCH₃, and -OCH₂CH₃; (C₁-C₃)-alkylphenyl; (C₁-C₃)-alkylphenyl, being the phenyl moiety substituted by at least a radical selected from the group consisting of CF₃, F, Cl, Br, I, CN, OH, -CH₃, -CH₂CH₃, -OCH₃, and -OCH₂CH₃;
or alternatively R₈ is a radical selected from pyrrolyl, furanyl, thiophenyl, pyrazolyl, imidazolyl, triazolyl, tetrazolyl, oxazolyl, isoxazolyl, thiazolyl, isothiazolyl, dithiolanyl, pyridinyl, pyranyl, pyridazinyl, pyrimidinyl, pyrazinyl, piperazinyl, benzofuranyl, isobenzofuranyl, indolyl, indenyl, dihydroindenyl, naphthalenyl, isoindolyl, indolizinyl, indolinyl, isoindolinyl, adeninyl, guaninyl, benzimidazolyl, indazolyl, benzoxazolyl, benzisoxazolyl, benzodioxolyl, benzofurazanyl, benzotriazolyl, benzothiofuranyl, benzothiazolyl, benzothiadiazolyl, chromenyl, isochromenyl, chromanyl, isochromanyl, benzodioxanyl, quinolinyl, and isoquinolinyl; being each ring forming part of the ring system optionally substituted by at least one radical selected from the group consisting of H; OH; -CH₂-NH-CH₂CCH; -NH-CH₂CCH; -CH₂-N(C₁-C₄)alkylCH₂CCH; - N(C₁-C₄)alkylCH₂CCH;linear or branched (C₁-C₆)alkyl; hydroxy-(C₁-C₄)alkyl; CF₃; CN; -O(C₁-C₄)alkyl; (C₁-C₄)alkyl-O-(C₁-C₄)alkyl; -C(O)H; -C(O)(C₁-C₄)alkyl; -COOH; -COO(C₁-C₄)alkyl; - OC(O)H; -OC(O)(C₁-C₄)alkyl; -C(O)NHCH₃; -C(O)N(CH₃)₂; -NH₂; -NHCH₃; - N(CH₃)₂; -CH₂NH₂; -CH₂CH₂NH₂; -CH₂NHCH₃; - CH₂N(CH₃)₂; -CH₂CH₂N(CH₃)₂; -CH₂N(CH₂CH₃)₂; -CH₂CH₂N(CH₂CH₃)₂; -SH; -SCH₃; -SCH₂CH₃; -SO-CH₃; -SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; - C=NH; -C=NCH₃; -C=NCH₂CH₃; -NCH₃C(O)CH₃; -NCH₃C(O)CH₂CH₃; - N=CH₂; -N=C(CH₃)₂; -N=C(CH₂CH₃)₂;
wherein n is an integer from 0 to 1,
and the B-A moiety is selected from the group consisting of B-(CH₂)ₙ₁-O-(CH₂)ₙ₂-; B-(CH₂)ₙ₁-NH-(CH₂)ₙ₂-; B-(CH₂)ₙ₁-CH₂-; B-(CH₂)ₙ₁-O-; B-(CH₂)ₙ₁-NH-; B-O-(CH₂)ₙ₂-; B-NH-(CH₂)ₙ₂-; B-; B-O-; B-NH-; B-O-(CH₂)ₙ₃-O-; B-O-(CH₂)ₙ₃-NH-; B-NH-(CH₂)ₙ₃-NH-; and B-NH-(CH₂)ₙ₃-O-;
and n1, n2 and n3 are an integer each independently selected from 1 to 4.

In another preferred embodiment, the invention relates to the compounds of formula (Ia) wherein

R₁ is selected from OH; -NH₂; -CN; -C(O)CH₃; -COOCH₃; -OC(O)CH₃; methylphenyl, being the phenyl moiety substituted by at least a radical selected from the group consisting of OH, halogen, methyl, ethyl, methoxy, and CN;

R₂ is selected from H; OH; -CH₂-NH-CH₂CCH; -NH-CH₂CCH; methyl; ethyl; -CH₂OH; -NH₂; -CN; -C(O)CH₃; -COOCH₃; -OC(O)CH₃; and -OCONH₂;

B is a radical of formula (VII), wherein R₈ is selected from the group consisting of H; OH; -CH₂-NH-CH₂CCH; -NH-CH₂CCH; methyl; ethyl; - CH₂OH; -NH₂; -CN; -C(O)CH₃; -COOCH₃; -OC(O)CH₃; -OCONH₂; phenyl; phenyl substituted by at least a radical selected from the group consisting of CF₃, F, Cl, Br, I, CN, OH, -CH₃, -CH₂CH₃, -OCH₃, and -OCH₂CH₃; (C₁-C₃)-alkylphenyl; (C₁-C₃)-alkylphenyl, being the phenyl moiety substituted by at least a radical selected from the group consisting of CF₃, F, Cl, Br, I, CN, OH, -CH₃, -CH₂CH₃, -OCH₃, and -OCH₂CH₃;
wherein n is an integer from 0 to 1,
the B-A moiety is selected from the group consisting of B-(CH₂)ₙ₁-O-(CH₂)ₙ₂-; B-(CH₂)ₙ₁-NH-(CH₂)ₙ₂-; B-(CH₂)ₙ₁-CH₂-; B-(CH₂)ₙ₁-O-; B-(CH₂)ₙ₁--NH-; B-O-(CH₂)ₙ₂-; B-NH-(CH₂)ₙ₂-; B-; B-O-; B-NH-; B-O-(CH₂)ₙ₃-O-; B-O-(CH₂)ₙ₃-NH-; B-NH-(CH₂)ₙ₃-NH-; and B-NH-(CH₂)ₙ₃-O-;
and n1, n2 and n3 are an integer each independently selected from 1 to 4.

More preferred are those compounds of formula (Ia) wherein
R₁ is selected from OH; -NH₂; -CN; -C(O)CH₃; -COOCH₃; and -OC(O)CH₃;
R₂ is selected from H; OH; and -CN;
B is a radical of formula (VII), wherein R₈ is selected from the group consisting of H; OH; methyl; ethyl; -CH₂OH; -NH₂; - CN; -C(O)CH₃; -COOCH₃; -OC(O)CH₃; -OCONH₂; phenyl; and benzyl;
wherein n is an integer from 0 to 1,
the B-A moiety is selected from the group consisting of B-(CH₂)ₙ₁-O-(CH₂)ₙ₂-; B-(CH₂)ₙ₁-NH-(CH₂)ₙ₂-; B-(CH₂)ₙ₁-CH₂-; B-(CH₂)ₙ₁-O-; B-(CH₂)ₙ₁-NH-; B-O-(CH₂)ₙ₂-; B-NH-(CH₂)ₙ₂-; B-; B-O-; B-NH-; B-O-(CH₂)ₙ₃-O-; B-O-(CH₂)ₙ₃-NH-; B-NH-(CH₂)ₙ₃-NH-; and B-NH-(CH₂)ₙ₃-O-;
and n1, n2 and n3 are an integer each independently selected from 1 to 2.

In a particularly preferred embodiment of this first aspect, the invention relates to the compounds of formula (I) which are selected from:
2-((1-Benzylpiperidin-4-yl)-2-(((8-hydroxyquinolin-5-yl)methyl)(prop-2-ynyl)amino)acetonitrile **(2);**
5-(((((1-Benzylpiperidin-4-yl)methyl)(prop-2-ynyl)amino)methyl)quinolin-8-ol **(3);**
3-((1-Benzylpiperidin-4-yl)-2-(((8-hydroxyquinolin-5-yl)methyl)(prop-2-ynyl)amino)propanenitrile **(4);**
5-((((2-(1-Benzylpiperidin-4-yl)ethyl)(prop-2-ynyl)amino)methyl)quinolin-8-ol **(5);**
4-((1-Benzylpiperidin-4-yl)-2-(((8-hydroxyquinolin-5-yl)methyl)(prop-2-yny)amino)butanenitrile **(6);**
5-((((3-(1-Benzylpiperidin-4-yl)propyl)(prop-2-ynyl)amino)methyl)quinolin-8-ol **(7).**

The compounds of the present invention are multifunctional compounds, comprising at least one residue that imparts both antiapoptotic and neuroprotective functions to the compound, at least one residue that imparts brain MAO inhibition function to the compound, and at least one residue that imparts biometal chelation function to the compound.

Therefore the compounds of the present invention are useful for treatment and/or prevention of diseases, disorders and conditions mediated by inhibition of amino oxidases (MAO), inhibition of cholinesterases (AChE, BuChE) and biometal chelation. In a particular embodiment the compounds of the invention are useful for the treatment and/or prevention of a neurodegenerative disorder, preferably such neurodegenerative disorder is selected from Alzheimer's disease, Parkinson's disease and neurodegeneration with brain iron accumulation (NBIA).

The "prevention" aspect of the use of the compounds of the invention in diseases such as Parkinson's disease and AD involves the prevention of further neurodegeneration and of the further progress of the disease.

One of the key pathological features of AD is cerebral amyloid angiopathy (CAA). CAA is present in most cases of AD, and it is characterized by the deposition of Aβ in brain vessels, inducing the degeneration of vascular smooth muscle cells and endothelial cells. Semicarbazide-sensitive amine oxidase (SSAO) is overexpressed in cerebrovascular tissue of patients with AD-CAA, and that it colocalizes with Aβ deposits. This over-expression correlates with high SSAO activity in plasma of severe AD patients. In addition, the catalytic activity of SSAO is able to induce apoptosis in smooth muscle cells in vitro, contributing to the vascular damage associated to AD. Accordingly, in order to evaluate the activity of the compounds of the present invention, it was determined radiochemically their ability to inhibit semicarbazide sensitive amine oxidase (SSAO).

The active agents useful in conventional treatment of a disease, condition and/or disorder mediated by inhibition of amino oxidases (MAO) and/or inhibition of cholinesterases (AChE, BuChE) and/or biometal chelation are preferably rivastigmine, donepezil, galanthamine and memantine.

Furthermore, the present invention covers all possible combinations of particular and preferred embodiments described hereinabove.

When the compounds according to the present invention are in the form of salts, they are preferably pharmaceutically acceptable salts. The term "pharmaceutically acceptable salt", as used herein, refers to salts derived from organic and inorganic acids. The compound of the general formula (I) may be converted into its pharmaceutically acceptable salts, or its pharmaceutically acceptable solvates by conventional methods. For example, such salts may be prepared by treating one or more of the compounds with an aqueous solution of the desired pharmaceutically acceptable metallic hydroxide or other metallic base and evaporating the resulting solution to dryness, preferably under reduced pressure in a nitrogen atmosphere. Alternatively, a solution of the compound of formula (I) may be mixed with an alkoxide of the desired metal, and the solution subsequently evaporated to dryness. The pharmaceutically acceptable hydroxides, bases, and alloxides include those worth cations for this purpose, including (but not limited to), potassium, sodium, ammonium, calcium, and magnesium. Other representative pharmaceutically acceptable salts include hydrochloride, hydrobromide, sulphate, bisulphate, lactate, phosphate, tosylate, citrate, maleate, fumarate, succinate, tartrate, acetate, oxalate, propionate, nitrate, methanesulfonate, benzoate and similarly known acceptable acids.

In addition to salt forms, the present invention provides compounds which are in a prodrug form. Prodrugs of the compounds described herein are those compounds which, upon administration to a subject, undergo chemical conversion by metabolic or chemical processes to yield a compound of formula (I), and/or a salt and/or solvate thereof. A prodrug is a pharmacologically active or inactive compound that is modified chemically through in vivo physiological action, such as hydrolysis, metabolism and the like, into a compound of this invention following administration of the prodrug to a patient. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme. The suitability and techniques involved in making and using prodrugs are well known by those skilled in the art.

It is clear to a person skilled in the art that certain compounds of the present invention possess asymmetric carbon atoms (optical centers) and/or double bonds. Accordingly, all stereoisomers of the compounds referred to herein, and mixtures thereof, such as those, for example, which may exist due to asymmetric carbons on any of the substituents, including enantiomeric forms (which may exist even in the absence of asymmetric carbons) and diastereomeric forms, are contemplated and within the scope of the preferred embodiments. Also individual stereoisomers which may, for example, be substantially free of other isomers, or may be admixed, for example, as racemates or with all other or other selected, stereoisomers are intended to be within the scope of the present invention. Thus, any given compound referred to herein is intended to represent any one of a racemate, one or more enantiomeric forms, one or more atropoisomeric forms, and mixtures thereof.

It should be noted that the general procedures are shown as it relates to preparation of compounds having unspecified stereochemistry. However, such procedures are generally applicable to those compounds of a specific stereochemistry, e.g., where the stereochemistry at a sterogenic center is (S) or (R). In addition, the compounds having one stereochemistry (e.g., (R)) can often be utilized to produce those having opposite stereochemistry (i.e., (S)) using well known methods, for example, by inversion. The same applies for Z/E isomers. Therefore, the preparation processes described herein can be modified to give enantiopure compounds as well as mixtures of stereoisomers. It is possible to prepare specific stereoisomers or specific mixtures by various processes including the use of stereospecific reagents or by introducing chiral centers into the compounds during its preparation process. In addition, it is possible to separate stereoisomers once the compound has been prepared by standard resolution techniques known to the skilled person.

The compounds of formula (I) may be in crystalline form either as free solvation compounds or as solvates (e.g. hydrates) and it is intended that both forms are within the scope of the present invention. Methods of solvation are generally known within the art.

Certain compounds of the present invention may exist in multiple crystalline or amorphous forms. In general, all physical forms are equivalent for the uses contemplated by the present invention and are intended to be within the scope of the present invention.

The present invention also relates to a pharmaceutical composition that comprises a compound of the present invention (or a pharmaceutically acceptable salt thereof) according to formula II and one or more pharmaceutically acceptable carriers. The carriers must be "pharmaceutically acceptable" in the sense of being compatible with the other ingredients of the composition and not deleterious to the recipients thereof.

Suitable carriers, excipients, etc. can be found in standard pharmaceutical texts.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration. The person skilled in the art will establish, considering the available knowledge, the necessary parameters and excipients.

In therapeutic use, the dosage may be varied depending upon the requirements of the patient, the severity of the condition being treated, and the compound being employed. Determination of optimus dosages for a particular situation is within the skill of the art.

The compounds of the present invention may be used in a substantially similar manner to other known therapeutic agents for treating the conditions or disorders above mentioned. The dose to be administered, whether a single dose, multiple dose, or a daily dose, will of course vary with the particular compound employed, the chosen route of administration, the body weight and body surface of the recipient, and the patient's physical condition and disease stage, among other factors. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological or physiological effects. A skilled physician in the art will be able to ascertain, appropriate protocols for the effective administration of the compounds of the preferred embodiments.

Also encompassed within the scope of the invention is a process for the preparation of a compound of formula (I) as described above.

The compounds of formula (I), where R₂=H, and R₁, n, A and B are as defined above, can be prepared by reaction of aldehydes of formula (IX) with propargylamine in the presence of sodium cyanoborohydride (NaCNBH₃), followed by reaction with the 5-(chloromethyl)quinolin-8-yl derivative of formula (X)

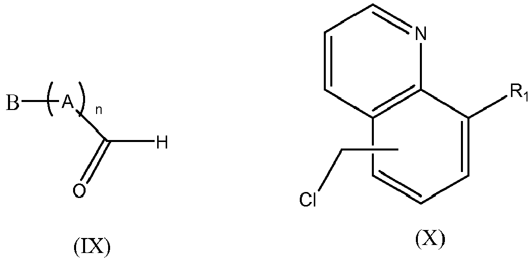

Alternatively, compounds of formula (I), where R₂=H, and R₁, n, A and B are as defined above, can be prepared starting from the 5-(chloromethyl)quinolin-8-yl derivative of formula (X), by reaction with propargylamine to give the 5-((prop-2-yn-1-ylamino)methyl)quinolin-8-yl derivative of formula (XI), followed by reaction with aldehydes of formula (IX) in the presence of sodium cyanoborohydride (NaCNBH₃)

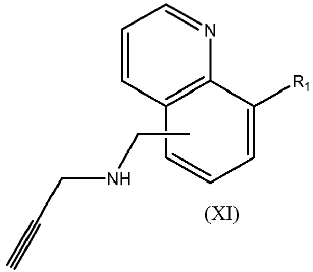

Compounds of formula (I), where R₂= CN, CO₂R₃, SO₂(4-MeC₆H₄) can be synthesized by reaction of aminoacetonitrile, glycine ethyl ester hydrochloride, and 1-[(4-methylphenyl)sulfonyl]methanamine, with (chloromethyl)quinolin-8-yl derivative of formula (X), respectively, to give compounds of formula (XII), followed by reaction with propargylamine to provide intermediates of type (XIII), and reaction with chlorides of general structure (XIV).

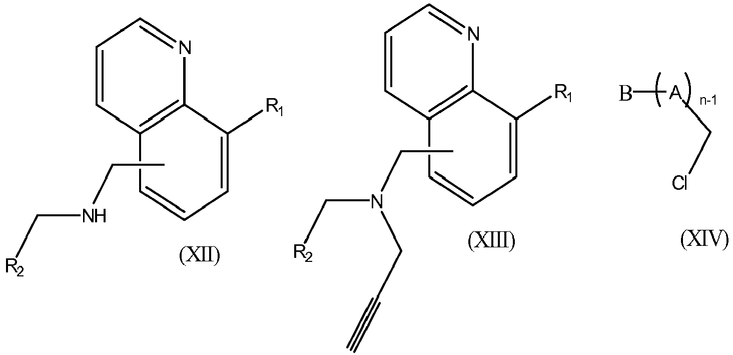

### DEFINITIONS

Unless otherwise defined, all technical and scientific terms used herein have the same meaning as commonly understood by one of ordinary skill in the art to which this invention belongs.

The term "MAO responsive disorder" and variations thereof refers to a disease or condition in which the biological function of a MAO inhibitor affects the development and/or course of the disease or condition, and/or in which modulation of MAO alters the development, course, and/or symptoms of the disease or condition. Modulation (e.g. inhibition) of the level of activity or MAO in a subject having a MAO responsive disorder may reduce the severity and/or duration of the disease, reduces the likelihood, prevents or delays the onset of the disease or condition, and/or causes an improvement in one or more symptoms of the disease or condition.

The term "ChE responsive disorder" and variations thereof refers to a disease or condition in which the biological function of a MAO inhibitor affects the development and/or course of the disease or condition, and/or in which modulation of ChE alters the development, course, and/or symptoms of the disease or condition. Modulation (e.g. inhibition) of the level of activity or ChE in a subject having a ChE responsive disorder may reduce the severity and/or duration of the disease, reduces the likelihood, prevents or delays the onset of the disease or condition, and/or causes an improvement in one or more symptoms of the disease or condition.

Throughout the present specification, by the term "treatment" is meant eliminating, reducing or ameliorating the cause, the effects or progression of a condition; and includes a reduction in the rate of progress, a halt in the rate of progress, amelioration of the condition, and cure of the condition. Treatment as a prophylactic measure (e.g., prophylaxis) is also included. The term "treatment" includes combination treatments and therapies, in which two or more treatments or therapies are combined, for example, sequentially or simultaneously. For purposes of this invention treatment includes, but is not limited to, alleviation, amelioration or elimination of one or more symptoms of the condition; diminishment of the extent of the condition; stabilized (i.e. not worsening) state of condition; delay or slowing of condition progression; amelioration or palliation of the condition state; and remission of the condition (whether partial or total).

The treatment defined hereinbefore may be applied as a sole therapy or may involve, in addition to the compound of the invention, one or more active agents useful in conventional treatment of such a disease, condition and/or disorder.

The expresions "combined therapy" refers to pharmaceutical preparations which are applied by simultaneously, sequentially or separately administrate at least one compound of formula (I) as defined above and one or more active agents useful in conventional treatment of such a disease, condition and/or disorder..

The term "known ring system" refers to a ring system which is known in the art and so intends to exclude those ring systems that are not chemically possible.

According to the present invention, a ring system formed by "isolated" rings means that the ring system is formed by two, three or four rings and said rings are bound via a bond from the atom of one ring to the atom of the other ring.

The term "isolated" also embraces the embodiment in which the ring system has only one ring. Illustrative non-limitative examples of known ring systems consisting of one ring are those derived from: cyclopropyl, cyclobutyl, cyclopentyl, cyclohexyl, cycloheptyl, cyclopropenyl, cyclobutenyl, cyclopentenyl, phenyl, cycloheptenyl, aziridinyl, oxirenyl, thiiranyl, azetidinyl, oxetanyl, pyrrolyl, furanyl, thiophenyl, pyrazolyl, imidazolyl, triazolyl, tetrazolyl, oxazolyl, isoxazolyl, thiazolyl, isothiazolyl, dithiolanyl, pyridinyl, pyranyl, pyridazinyl, pyrimidinyl, pyrazinyl, piperazinyl, oxazinyl, thiazinyl, dithianyl, and dioxanyl.

According to the present invention, a ring system having rings "totally fused" means that the ring system is formed by two, three or four rings in which two or more atoms are common to two adjoining rings. Illustrative non-limitative examples of known ring systems consisting of two rings totally fused, are those derived from benzofuran, isobenzofuran, indole, indene, dihydroindene, naphthalene, isoindole, indolizine, indoline, isoindoline, purine (e.g., adenine, guanine), benzimidazole, indazole, benzoxazole, benzisoxazole, benzodioxole, benzofurazan, benzotriazole, benzothiofuran, benzothiazole, benzothiadiazole, heterocyclic chromene, isochromene, chroman, isochroman, benzodioxan, quinoline, isoquinoline, quinolizine, benzoxazine, benzodiazine, pyridopyridine, quinoxaline, quinazoline, cinnoline, phthalazine, naphthyridine, pteridine. Illustrative non-limitative examples of known ring systems consisting of 3 fused rings are carbazole, dibenzofuran, dibenzothiophene, carboline, perimidine, pyridoindole, acridine, xanthene, thioxanthene, oxanthrene, phenoxathiin, phenazine, phenoxazine, phenothiazine, thianthrene, phenanthridine, phenanthroline, phenazine.

According to the present invention when the ring system is "partially fused" it means that the ring system is formed by three or four rings, being at least two of said rings totally fused (i.e. two or more atoms being common to the two adjoining rings) and the remaining ring(s) being bound via a bond from the atom of one ring to the atom of one of the fused rings.

In the present invention, the term "alkene" is to be understood as an unsaturated chemical compound containing at least one carbon-to-carbon double bond. Preferably, the alkene is selected from the group consisting of: 1,3-dienes, 1,3,5-trienes, terminal alkenes and internal alkenes.

The term "(C₁-C₆)alkyl" as used herein refers to a saturated branched or linear hydrocarbon chain with 1 to 6 hydrocarbon atoms. Preferably "(C₁-C₆)alkyl" is an unsubstituted group selected from methyl, ethyl, propyl, isopropyl, butyl, isobutyl, s-butyl and t-butyl.

The term "(C₁-C₆)-alkoxy" as used herein refers to a saturated branched or linear hydrocarbon chain with 1 to 6 hydrocarbon atoms (i.e. (C₁-C₆)alkyl groups as defined above) linked to an oxygen, thus (C₁-C₆)alkyl-O. Preferably "(C₁-C₆)-alkoxy" is an unsubstituted group selected from methoxy, ethoxy, propoxy, isopropoxy, butoxy, isobutoxy, s-butoxy, and t-butoxy.

The term "halogen" is meant to include fluorine, chlorine, bromine and iodine.

The term "pharmaceutically acceptable" as used herein pertains to compounds, materials, compositions, and/or dosage forms which are, within the scope of sound medical judgment, suitable for use in contact with the tissues of a subject (e. g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

The term "therapeutically effective amount," as used herein, pertains to that amount of an active compound, or a material, composition or dosage form comprising an active compound, which is effective for producing some desired therapeutic effect.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

Melting points were determined on a Koffler apparatus, and are uncorrected. ¹H NMR and ¹³C NMR spectra were recorded in CDCl₃ or DMSO-d₆ at 300, 400 or 600 MHz and at 100 or 150 MHz, respectively, using solvent peaks [CDCl₃: 7.27 (*D*), 77.2 (C) ppm; D₂O: 4.60 ppm and DMSO-d₆: 2.49 (*D*), 40 (C) ppm] as internal reference. The assignment of chemical shifts is based on standard NMR experiments (¹H, ¹³C-DEPT, ¹H, ¹H-COSY, gHSQC, gHMBC). Mass spectra were recorded on a GC/MS spectrometer with an API-ES ionization source. Elemental analyses were performed at CNQO (CSIC, Madrid, Spain). TLC were performed on silica F254 and detection by UV light at 254 nm or by charring with either ninhydrin, anisaldehyde or phosphomolybdic-H₂SO₄ dyeing reagents. Anhydrous solvents were used in all experiments. Column chromatography was performed on silica gel 60 (230 mesh).

### Example 1. Synthesis of intermediate 5-(chloromethyl)quinolin-8-ol (9).

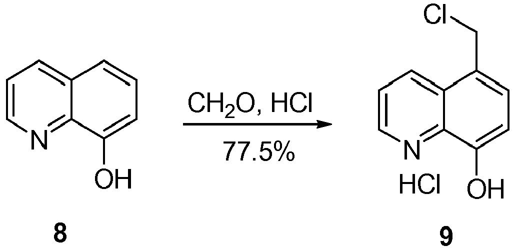

To a cooled solution of comercial 8-hydroxyquinoline (14.6 g, 100 mmol) in conc. HCl (44 mL) at 0 °C, a 37% aqueous formaldehyde solution (20 mL) was added. Then HCl gas was bubbled through the solution with stirring for 2 h. The mixture was allowed to warm to rt with further stirring for 6 h and without stirring for 2 h more. The product was filtered and the solid was rinsed with conc. HCl, giving product **9** (19.9 g, 77.5%) as light yellow solid: ¹H NMR (300 MHz, DMSO-*d*₆) δ 5.30 (s, 2H), 7.51 (d, *J*= 8.0 Hz, 1H), 7.86 (d, *J*= 8.0 Hz, 1H), 8.13 (dd, *J*= 8.7, 5.2 Hz, 1H), 9.12 (dd, *J*= 5.1, 1.3 Hz, 1H), 9.24 (dd, *J*= 8.8, 1.3 Hz, 1H).

### Example 2. Synthesis of intermediates N-r(1-benzylpiperidin-4-yl)methyl]prop-2-yn-1-aminen (10) and (1-benzylpiperidin-4-yl)(prop-2-yn-1-ylamino)acetonitrile (11).

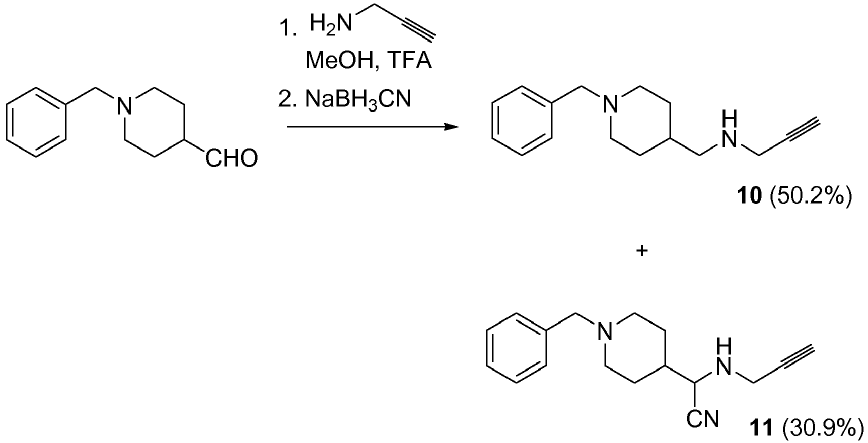

To a cooled solution of 1-benzyl-4-piperidinecarboxaldehyde (407 mg, 2.0 mmol) and propargylamine (165 mg, 3.0 mmol) in MeOH (6 mL) at 0 °C, a small amount of CF₃CO₂H (5 drops) was added. After being stirred for 1 h, NaBH₃CN (189 mg, 2.9 mmol) was added to the solution portionwsise. The mixture was stirred at rt overnight and then quenched with aqueous saturated NaHCO₃. The mixture was concentrated in vacuo and then extracted with AcOEt. The combined organic layers were dried over MgSO₄ and concentrated under reduced pressure to give a mixture that was purified by column chromatography (SiO₂, eluting solvent was changed from hexane/AcOEt= 5:1 to AcOEt and then AcOEt/MeOH= 4:1 v/v) to give compound **10** (232 mg, 50.2%), as an orange oil, and product **11** (165 mg, 30.9%) as an orange oil. N-[(1-benzylpiperidin-4-yl)methyl]prop-2-yn-1-amine **(10):** *R_{f}=* 0.23 (AcOEt/MeOH= 4:1); IR (film) ν 3287, 3028, 2918, 2801, 2758, 1495, 1454, 1366, 1342, 1121, 1078 cm⁻¹; ¹H NMR (400 MHz, CDCl₃) δ 1.15-1.55 (m, 3 H), 1.72 (d, *J*= 12.5 Hz, 2 H), 1.95-2.11 (m, 2 H), 2.20 (t, *J*= 2.3 Hz, 1 H), 2.57 (d, *J*= 6.6 Hz, 2 H), 2.94 (d, *J*= 11.7 Hz, 2 H), 3.33-3.65 (m, 4 H), 7.19-7.40 (m, 5H ); ¹³C NMR (100 MHz, CDCl₃) δ 30.1, 35.8, 38.4, 53.4, 54.5, 63.1, 71.4, 82.2, 127.3, 128.3, 129.4, 137.3. (1-Benzylpiperidin-4-yl)(prop-2-yn-1-ylamino) acetonitrile **(11):** *R_{f}=* 0.78 (AcOEt/MeOH= 4:1); IR (film) ν 3296, 2940, 2920, 2803, 2760, 1732, 1452, 1368, 1246, 1223, 1072 cm⁻¹; ¹H NMR (400 MHz, CDCl₃) δ 1.37-1.72 (m, 4 H), 1.76-1.88 (m, 2 H), 1.92-2.02 (m, 2 H), 2.26-2.33 (m, 1 H), 2.87-2.98 (m, 2 H), 3.44-3.69 (m, 5 H), 7.17-7.44(m, 5 H); ¹³C NMR (100 MHz, CDCl₃) δ 28.4, 29.0, 36.8, 39.0, 53.0, 54.5, 63.0, 72.8, 80.1, 118.7, 127.1, 128.2, 129.1, 138.3.

### Example 3. Synthesis of 5-((((1-benzylpiperidin-4-yl)methyl)(prop-2-ynyl)amino)methyl)quinolin-8-ol (3).

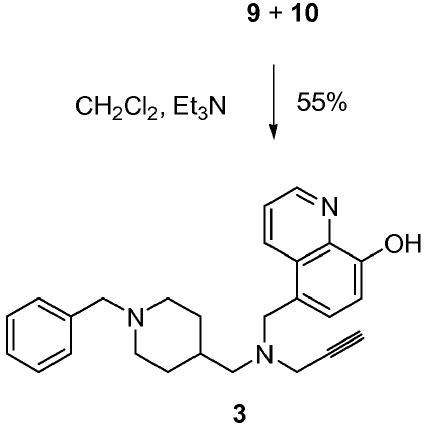

To a stirring solution of compound 10 (638 mg, 2.6 mmol) and chloride **9** (694 mg, 2.7 mmol) in CH₂Cl₂ (12 mL), Et₃N (1.09 mL, 5.2 mmol) was added at rt. After being stirred overnight, the mixture was quenched with water and the product was extracted with CH₂Cl₂, dried over Mg₂SO₄, concentrated in vacuo, and purified by column chromatography (SiO₂, hexane/AcOEt by increasing the gradient from 5:1 to 1:1 v/v) to give amine 3 (572 mg, 55%) as a light brown solid: mp 128.5 °C; IR (KBr) ν 3320, 3248, 2946, 2914, 2754, 2365, 1734, 1580, 1506, 1478, 1422, 1366, 1279, 1229, 1192, 1128, 1065 cm⁻¹; ¹H NMR (400 MHz, CDCl₃) δ 0.97-1.14 (m, 2H), 1.32-1.48(m,1 H), 1.60 (d, *J*= 12.5 Hz, 2 H), 1.84 (t, *J*= 11.0 Hz, 2 H), 2.19 (s, 1 H), 2.36 (d, *J*= 7.2 Hz, 2H), 2.76 (d, *J*= 11.1 Hz, 2 H), 3.15 (d, *J*= 1.9 Hz, 2 H), 3.39 (s, 2 H), 3.83 (s, 2 H), 6.98 (d, *J*= 7.8 Hz, 1 H), 7.12-7.37 (m, 7 H), 8.53 (dd, *J*= 8.5, 1.4 Hz, 1 H), 8.68 (dd, *J*= 4.2, 1.4 Hz, 1 H); ¹³C NMR (100 MHz, CDCl₃) δ 30.8, 33.5, 40.8, 53.6, 56.6, 59.1, 63.5, 73.4, 78.4, 108.7, 121.3, 124.9, 126.9, 127.8, 128.1, 129.1, 129.2, 134.1, 138.5, 138.7, 147.5, 151.9. Anal. Calcd for C₂₆H₂₉N₃O: C, 78.16; H, 7.32; N,10.52. Found; C, 78.05; H, 7.36; N,10.40.

### Example 4. Synthesis of 2-(1-benzylpiperidin-4-yl)-2-(((8-hydroxyquinolin-5-yl)methyl)(prop-2-ynyl)amino)acetonitrile (2).

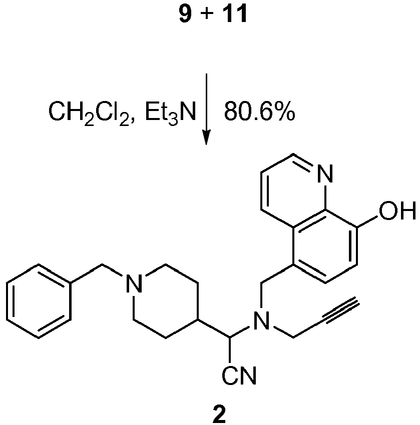

To a solution of compound 11 (70 mg, 0.29 mmol) and Et₃N (0.085 mL, 0.58 mmol) in CH₂Cl₂ (2 mL), chloride 9 (70 mg, 0.25 mmol) was added. After stirring for 30 min at rt, brine was added to the mixture. The product was extracted with CH₂Cl₂, the extract was dried over Mg₂SO₄ and then concentrated in vacuo. The crude product was purified by column chromatography (hexane/AcOEtby increasing the gradient from 5:1 to 1:1 v/v), to give compound 2 (99 mg, 80.6%) as a white solid: IR (KBr) ν 3443, 3300, 3026, 2949, 2814, 1580, 1504, 1476, 1454, 1424, 1371, 1269, 1229, 1193, 1150, 1072 cm⁻¹; ¹H NMR (600 MHz, CDCl₃) δ 0.68-0.70 (m, 1 H), 1.17-1.22 (m, 1 H), 1.65-1.95 (m, 5 H), 2.41 (s, 1 H), 2.65 (s, 1H), 2.81-2.83 (m, 1 H), 3.23-3.29 (m, 2 H), 3.34 (s, 1 H), 3.45-3.48 (m, 1 H), 3.68 (d, *J*= 13.2 Hz, 1 H), 4.63 (d, *J*= 13.8 Hz, 1 H), 7.1-7.20 (m, 1 H), 7.21-7.28 (m, 5 H), 7.40-7.44 (m, 2 H), 8.60 (d, *J*= 8.4 Hz, 1 H), 8.76 (d, *J*= 4.2 Hz, 1 H); ¹³C NMR (150 MHz, CDCl₃) δ 29.4, 30.7, 36.0, 40.0, 52.5, 54.2, 57.7, 63.1, 74.0, 78.8, 84.7, 108.9, 111.8, 116.0, 121.7, 122.2, 127.2, 127.5, 128.2, 129.3, 130.0, 134.0, 138.8, 148.9, 152.6. Anal. Calcd for C₂₇H₂₈N₃O: C, 76.39; H, 6.65; N,13.20. Found: C, 76.45; H, 6.75; N 13.17.

### Example 5. Synthesis of 5-(((2-(1-benzylpiperidin-4-yl)ethyl)(prop-2-ynyl)amino)methyl)quinolin-8-ol (5) and 3-(1-benzylpiperidin-4-yl)-2-(((8-hydroxyquinolin-5-yl)methyl)(prop-2-ynyl)amino)propanenitrile (4).

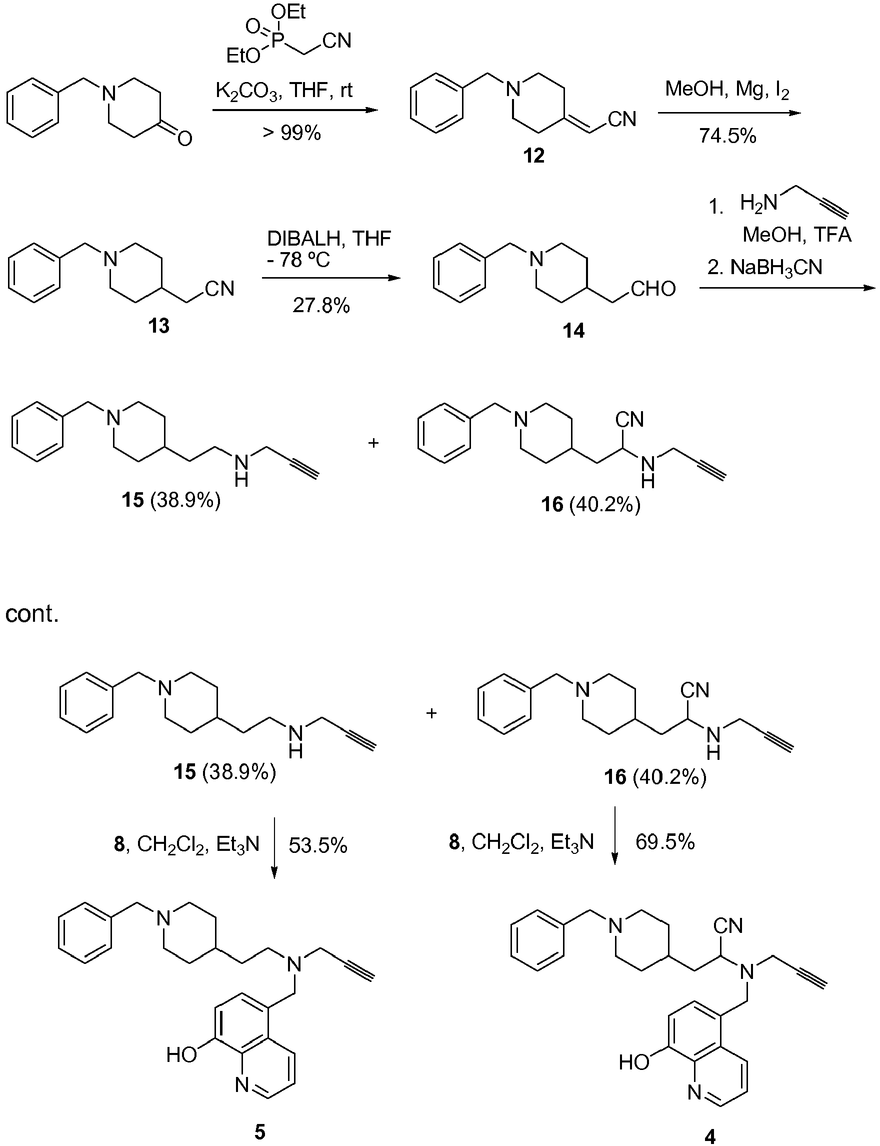

Diethyl(cyanomethyl)phosphonate. Diethyl(cyanomethyl)phosphonate was prepared by heating triethylphosphite (1.0 equiv) and chloroacetonitrile (1.0 equiv) at 150 °C for 3.5 h. The crude product was directly used in the next reaction.

(1-Benzylpiperidin-4-ylidene)acetonitrile (**12**) A solution of diethyl (cyanomethyl)phosphonate (2.13 g, 12 mmol) and K₂CO₃ (1.39 g, 10 mmol) in dry THF (5 mL) was stirred for 15 min at rt. Then the mixture was heated to reflux for 20 min. After cooling down to rt, 1-benzyl-4-piperidone (1.90 g, 10 mmol) was added dropwise to this solution. Then the mixture was heated at reflux for 12 h. After cooling down to rt, 10% K₂CO₃ aqueous solution was added. The reaction mixture was extracted with AcOEt, and the organic layers were dried over MgSO₄ and concentrated in vacuo to give the crude product **12** [2.7 g, >99.0 %; ¹H NMR (300 MHz, CDCl₃) δ 2.40 (t, *J*= 5.1 Hz, 2 H), 2.51-2.63 (m, 6 H), 3.55 (s, 2 H), 5.10 (s, 1 H), 7.26-7.34 (m, 5 H)], which was directly used in next step.

(1-Benzylpiperidin-4-yl)acetonitrile **(13)** To a solution of nitrile **12** (2.7 g, 10 mmol) in MeOH (100 mL), Mg (4.6 g, 191 mmol) and infinitesimal quantity of I₂ was added. The mixture was stirred until it became gray gel. After conc. HCl was added, the mixture became clear solution. Then it was treated with 10 N NaOH to alkaline. The precipitate was filtered and washed with large amount of EtOAc. The filtrate was extracted with AcOEt, and the combined organic layers were dried over MgSO₄ and concentrated in vacuo to give the product **13** (1.59 g, 74.5 %): ¹H NMR (300 MHz, CDCl₃) δ 1.48 (m, 2 H), 1.68 (m, 1 H), 1.78 (m, 2 H), 2.00 (t, *J* = 52.77 Hz, 2 H), 2.28 (d, *J* = 6.60 Hz, 2 H), 2.93 (d, *J*= 11.8 Hz, 2 H), 3.55 (s, 2 H), 7.24-7.34 (m, 5 H).

(I -Benzvloioeridin-4-_{V}l)acetaldehvde (**14**) To an oven-dried and argon-purged flask were added the nitrile **13** (1.29 g, 6.0 mmol) and THF (13 mL). The mixture was cooled to -78 °C, and DIBAL-H (6.22 mL, 1 mmol/mL) was added to the reaction via syringe. The reaction was stirred at -78 °C for 1 h, and then quenched with aqueous saturated NaHCO₃. The precipitate was filtered and washed with large amount of EtOAc. The filtrate was extracted with EtOAc. The combined organic layers were washed with brine and dried over MgSO₄. After concentrated in vacuum, the crude product was purified by chromatography (SiO₂, CH₂Cl₂/MeOH =20:1 v/v) to give product **10** (365 mg, 27.8%) as an orange oil: ¹H NMR (300 MHz, CDCl₃) δ 1.20-1.50 (m, 2 H), 1.60-2.15 (m, 5 H), 2.36 (dd, *J*= 6.7, 1.76 Hz, 2 H), 2.88 (d, *J*= 11.2 Hz, 2 H), 3.51 (s, 2 H), 7.17-7.42 (m, 5 H), 9.76 (t, *J*= 1.9 Hz, 1 H).

*N*-[2-(1-Benzylpiperidin-4-yl)ethyl]prop-2-yn-1-amine (**15**) and 3-(1-benzylpiperidin-4-yl)-2-(prop-2-yn-1-ylamino)propanenitrile (**16**). A solution of compound **14** (365 mg, 1.68 mmol) and propargylamine (187 mg, 3.4 mmol) in MeOH (10 mL) was stirred at 0 °C for 1 h; then NaBH₃CN (214 mg, 3.4 mmol) was added. The mixture was stirred at rt overnight. Water was added to the mixture and MeOH was removed under reduced pressure. Then aqueous saturated NaHCO₃ was added, and the mixture was extracted with AcOEt. The separated organic layers were dried over MgSO₄ and concentrated in vacuo to give the crude product. Further purification was achieved by column chromatography (SiO₂, eluting solvent was changed with gradient from hexane/AcOEt 5:1 to 1:5v/v and then change from AcOEt to AcOEt/MeOH 4:1 v/v) to give compound **15** (168 mg, 38.9%), as an orange oil, and product **16** (190 mg, 40.2%) as an orange oil. *N*-[2-(1-Benzylpiperidin-4-yl)ethyl]prop-2-yn-1-amine **(15):** *R_{f}*= 0.11 (MeOH/AcOEt= 1:4); IR (film) ν 3302, 2922, 2945, 2801, 2758, 1493, 1454, 1366, 1148, 1121, 1078, 1028 cm⁻¹; ¹H NMR (400 MHz, CDCl₃) δ 1.15-1.45 (m, 5H), 1.61 (d, *J*= 10.5 Hz, 2 H), 1.94 (t, *J*= 11.1 Hz, 2 H),2.13 (t, *J*= 2.1 Hz, 1 H),2.63 (t, *J*= 7.2 Hz, 2 H), 2.84 (d, *J*= 11.5 Hz, 2 H),3.30-3.55 (m, 4 H), 7.12-7.33 (m, 5 H); ¹³C NMR (100 MHz, CDCl₃) δ 32.1, 33.5, 36.4, 38.2, 46.0, 53.7, 63.3, 71.4, 82.2, 127.0, 128.1, 129.3,129.4, 137.6. 3-(1-Benzylpiperidin-4-yl)-2-(prop-2-yn-1-ylamino)propanenitrile **(16):** *R_{f}=* 0.56 (MeOH/AcOEt= 1:4); IR (film) ν 3295, 2924, 2905, 2782, 1493, 1452, 1388, 1343, 1125, 1074 cm⁻¹; ¹H NMR (400 MHz, CDCl₃) δ 1.15-1.38 (m, 2 H), 1.43-1.73 (m, 5 H), 1.92 (t, *J*= 11.5 Hz, 2 H), 2.21 (t, *J*= 2.3 Hz, 1 H), 2.82 (d, *J*= 10.7 Hz, 2 H), 3.35-3.64 (m, 4 H), 3.66-3.78 (m, 1H), 7.13-7.33 (m, 5 H); ¹³C NMR (100 MHz, CDCl₃) δ 31.7, 31.9, 32.2, 36.4, 39.9, 46.9 53.2, 63.2, 72.7, 79.8, 119.6, 126.9, 128.0, 129.0, 129.1, 138.1.

5-(((2-(1-Benzylpiperidin-4-yl)ethyl)(prop-2-ynyl)amino)methyl)quinolin-8-ol (**5**). To a cooled mixture of compound **15** (46 mg, 0.18 mmol) and Et₃N (0.053 mL, 0.38 mmol) in CH₂Cl₂ (2.5 mL) at 0 °C, chloride **8** (47 mg, 0.18 mmol) was added. After being stirred at rt overnight, the reaction was quenched with aqueous 5% NaHCO₃. The product was extracted with CH₂Cl₂, and extracts were dried over Mg₂SO₄ and then concentrated in vacuo. The crude product was purified by column chromatography (hexane/AcOE tby increasing the gradient from 5:1 to 1:1 v/v) to give compound **5** (40 mg, 53.5%) as a yellow oil: IR (film) □ ν 3254, 2945, 2915, 2805, 2758, 1578, 1508, 1478, 1420, 1375, 1350, 1279, 1231, 1194, 1148, 1121 cm⁻¹; ¹H NMR (400 MHz, CDCl₃) δ 1.24 (s, 3 H), 1.44 (d, *J* = 33.20 Hz, 4 H), 1.82 (m, 2 H), 2.67 (t, *J* = 2.40 Hz, 1 H), 2.58 (t, *J* = 7.20 Hz, 2 H), 2.83 (d, *J* =11.20 Hz, 2 H), 3.26 (d, *J* = 2.40 Hz, 2 H), 3.48 (s, 2 H), 3.92 (s, 2 H), 7.08 (d, *J =* 8.00 Hz, 1 H), 7.30-7.44 (m, 7 H), 8.61-8.61 (m, 1 H), 8.76-8.77 (m, 1 H); ¹³C NMR (100 MHz, CDCl₃) δ 32.2, 33.5, 33.9, 41.0, 50.3, 53.7, 55.9, 63.3, 73.3, 78.3, 108.7, 121.4, 124.9, 127.0, 127.9, 128.1, 129.2, 129.3, 134.0, 138.7, 147.5, 151.8. HRMS. Calcd for C₂₇H₃₂N₃O (MH+): 414.2545. Found: 414.2570 (MH+).

3-(1-Benzylpiperidin-4-yl)-2-(((8-hydroxyguinolin-5-yl)methyl)(prop-2-ynyl)amino)propanenitrile (**4**). To a cooled mixture of **16** (84 mg, 0.3 mmol) and Et₃N (0.093 mL, 0.67 mmol) in CH₂Cl₂ (6 mL), chloride **8** (77 mg, 0.3 mmol) was added at 0 °C. After being stirred at rt overnight, the reaction was quenched with aqueous 5% NaHCO₃. The reaction mixture was extracted with CH₂Cl₂, and extracts were dried with Mg₂SO₄ and concentrated in vacuo. The crude product was purified by column chromatography (hexane:AcOEt by increasing the gradient from 5:1 to 1:1 v/v) to give compound **4** (91.4 mg, 69.5%) as an orange oil: IR (film) ν 3298, 2934, 2807, 1580, 1505, 1476, 1370, 1273, 1233, 1198, 1148, 1072, 1028 cm⁻¹; ¹H NMR (400 MHz, CDCl₃) δ 0.81-0.93 (m, 1 H), 0.93-1.07 (m, 1 H), 1.12-1.32 (m, 2 H), 1.38-1.62 (m, 3 H), 1.69 (ddd, *J*= 14.1, 8.8, 5.5 Hz, 1 H), 1.76-1.89 (m, 1 H), 2.39-2.47 (m, 1 H), 2.58 (d, *J*= 11.3 Hz, 1 H), 2.80 (d, *J*= 11.5 Hz, 1 H), 3.21-3.32 (m, 1 H), 3.36-3.53 (m, 3 H), 3.65-3.75 (m, 2 H), 4.60 (d, *J*= 13.1 Hz, 1 H), 7.06-7.11 (m, 1 H), 7.21-7.33 (m, 5 H), 7.40-7.47 (m, 2 H), 8.58-8.65 (m, 1 H), 8.75-8.81 (m, 1 H); ¹³C NMR (100 MHz, CDCl₃) δ 30.6, 31.5, 37.2, 39.8, 49.6, 52.7, 53.0, 53.6, 62.8, 74.1, 78.6, 108.9, 117.0, 121.6, 122.3, 127.2, 127.5, 128.1,129.3, 129.8, 133.6, 138.6, 147.7, 152.5. HRMS. Calcd for C₂₉H₃₂N₄O (MH+): 439.2428. Found: 439.2532 (MH+).

### Example 6. Synthesis of 5-(((3-(1-benzylpiperidin-4-yl)propyl)(prop-2-ynyl)amino)methyl)quinolin-8-ol (7) and 4-(1-benzylpiperidin-4-yl)-2-(((8-hydroxyquinolin-5-yl)methyl)(prop-2-yny)amino)butanenitrile (6).

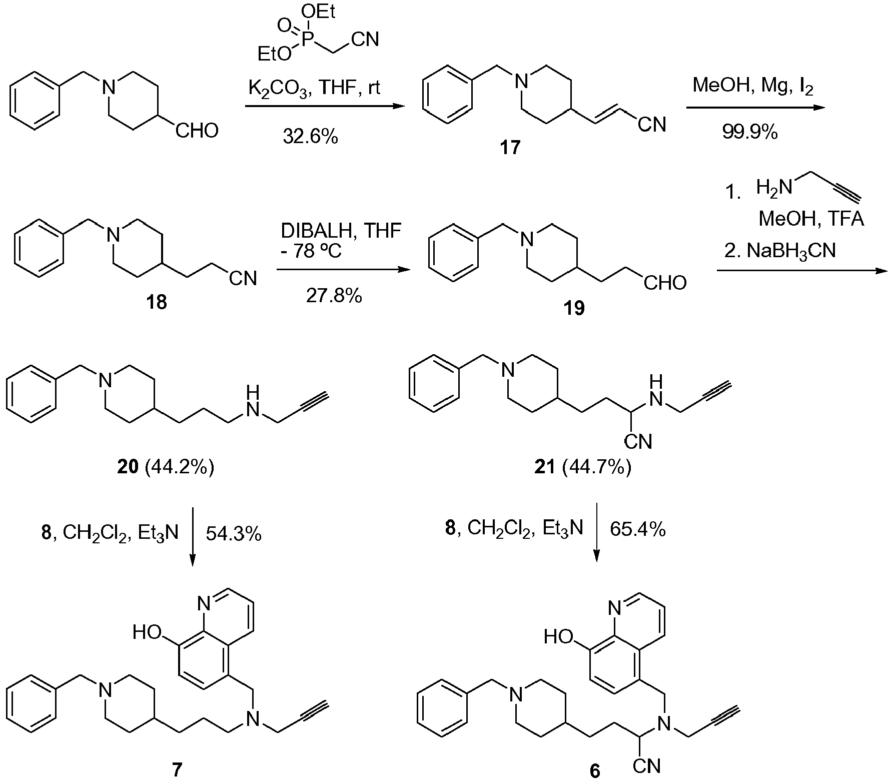

(2E)-3-(1-Benzylpiperidin-4-yl)prop-2-enenitrile **(17)** A mixture of diethyl (cyanomethyl)phosphonate (2.2 g, 12 mmol) and K₂CO₃ (1.4 g, 10 mmol) in dry THF (100 mL) was stirred at rt for 15 min, and then heated at reflux for 20 min. After cooling down to rt, 1-benzyl-4-piperidinecarboxaldehyde (2.0 g, 10 mmol) was added. The mixture was heated to reflux for 3 h. Aqueous 10% K₂CO₃ water (100 mL) was added after cooling down to rt. The product was extracted with AcOEt and dried over MgSO₄. After concentrated in vacuo, the crude product was purified by column chromatography (SiO₂, hexane/AcOEt from 5:1 to 1:1 v/v) to give nitrile **17** (1.0 g,44.5% yield, *R_{f}=* 0.70) and (0.74 g, 32.6%) as white solid: *R_{f}=* 0.30 (hexane/AcOEt= 1 :2); ¹H NMR (300 MHz, CDCl₃) δ 1.40-1.60 (m, 2 H), 1.62-1.77 (m, 2 H), 1.93-2.14 (m, 2 H), 2.51-2.71 (m, 1 H), 2.89 (d, *J*= 11.7 Hz, 2 H), 3.50 (s, 2 H), 5.23 (d, *J*= 10.9 Hz, 1 H), 6.31 (t, *J*= 10.4 Hz, 1 H), 7.19-7.38 (m, 5 H).

3-(1-Benzylpiperidin-4-yl)propanenitrile (**18**) To a solution of **17** (1.74 g, 7.70 mmol) in MeOH (33 mL) at rt, turning of Mg (3.70 g, 154 mmol) and infinitesimal quantity of I₂ was added to the mixture. The mixture was stirred until it became gray gel. After conc. HCl was added, the mixture became clear solution. Then it was treated with 10 N NaOH to alkaline. The precipitates were filtered and washed with large amount of EtOAc. The filtrate was extracted with AcOEt, and the combined organic layers were dried over MgSO₄ and concentrated in vacuo to give the crude product **18** (1.76 g, 99.9%) as an orange oil: ¹H NMR (300 MHz, CDCl₃) δ 1.17-1.52 (m, 3 H), 1.54-1.73 (m, 4 H), 1.96 (td, *J*= 11.5, 1.9 Hz, 2 H), 2.35 (t, *J*= 7.3 Hz, 2 H), 2.89 (d, J= 11.8 Hz, 2 H), 3.50 (s, 2 H), 7.19-7.37 (m, 5 H).

3-(1-Benzylpiperidin-4-yl)propanal (**19)** To an oven-dried and argon-purged flask were added the nitrile **18** (1.00 g , 4.4mmol) and THF (10 mL). The reaction was cooled to -78 °C, and DIBAL-H (4.54 mL, 1mmol/mL) was added to the reaction via syringe. The mixture was stirred at -78 °C for 1 h, and then quenched with aqueous saturated NaHCO₃. The precipitates were filtered and washed with large amount of EtOAc. The filtrate was extracted with EtOAc. The combined organic layers were washed with brine and dried over MgSO₄. After concentrated in vacuum, the crude product was purified by chromatography(SiO₂, CH₂Cl₂/ MeOH =20:1 v/v), to give aldehyde **19** (427 mg, 42.7%) as orange oil: ¹H NMR (400 MHz, CDCl₃) δ 1.16-1.35 (m, 3 H), 1.52-1.72 (m, 4 H), 1.92 (t, *J*= 11.8 Hz, 2 H), 2.43 (td, *J*= 7.5, 1.6 Hz, 2 H), 2.87 (d, *J*= 11.1 Hz, 2 H), 3.44-3.53 (m, 2 H), 7.19-7.38 (m, 5 H), 9.76 (t, *J*= 1.6 Hz, 1 H).

*N*-[3-(1-Benzylpiperidin-4-yl)propyl]prop-2-yn-1-amine (**20)** and 4-(1-benzylpiperidin-4-yl)-2-(prop-2-yn-1-ylamino)butanenitrile (**21**). A solution of aldehyde **19** (427 mg, 1.9 mmol) and propargylamine (204 mg, 3.7 mmol) in MeOH (6 mL) was stirred at 0 °C for 1 h, then NaBH₃CN (1.3 g, 2.0 mmol) was added. The mixture was stirred at rt overnight. Water was added to the mixture and MeOH was removed under reduced pressure. Then aqueous saturated NaHCO₃ was added, and the mixture was extracted with AcOEt. The separated organic layers were dried over MgSO₄ and concentrated in vacuo to give the crude products. Further purification was achieved by column chromatography (SiO₂, eluting solvent was changed with gradient from hexane/AcOEt 5:1 to 1:5v/v and then change from AcOEt to AcOEt/MeOH 4:1 v/v) to give products **20** (221 mg, 44.2%), as an yellow oil, and **21** (234 mg, 42.7%), as an yellow oil. *N*-[3-(1-Benzylpiperidin-4-yl)propyl]prop-2-yn-1-amine **20:** *R_{f}=* 0.19 (MeOH/AcOEt= 1:4); IR (film) ν 3304, 3028, 2922, 2847, 2794, 2758, 1495, 1452, 1386, 1343, 1119, 1028 cm⁻¹; ¹H NMR (600 MHz, CDCl₃) δ 1.10-1.26 (m, 5 H), 1.33-1.46 (m, 2 H), 1.58 (d, *J*= 9.9 Hz, 2 H), 1.87 (br s, 2 H), 2.13 (t, *J*= 2.3 Hz, 1 H), 2.31-2.39 (m, 1 H), 2.59 (t, *J*= 7.1 Hz, 1 H), 2.81 (d, *J*= 10.8 Hz, 2 H), 3.35 (d, *J*= 2.3 Hz, 2 H), 3.43 (s, 2 H), 7.13-7.29 (m, 5 H); ¹³C NMR (150 MHz, CDCl₃) δ 24.6, 27.0, 32.2, 32.3, 34.1, 35.6, 38.0, 48.8, 53.8, 63.4, 71.1, 82.2, 126.7, 128.0, 129.1, 138.4. 4-(1-Benzylpiperidin-4-yl)-2-(prop-2-yn-1-ylamino)butanenitrile **21:** *R_{f}=* 0.59 (MeOH/AcOEt= 1:4); IR (film) ν 3296, 2911, 2845, 2801, 2780, 1493, 1452, 1368, 1343, 1312, 1260, 1146, 1119, 1074; ¹H NMR (400 MHz, CDCl₃) δ 1.18-1.69 (m, 8 H), 1.70-1.83 (m, 2 H), 1.85-2.02 (m, 2 H), 2.23-2.37 (m, 1 H), 2.87 (d, *J*= 10.5 Hz, 2 H), 3.29-3.76 (m, 4 H), 7.16 -7.38 (m, 5 H); ¹³C NMR (100 MHz, CDCl₃) δ 31.0, 31.6, 32.1, 32.2, 32.3, 35.4, 36.6, 49.5, 53.7, 63.4, 72.8, 80.0, 119.4, 126.7, 127.9, 128.9, 138.1.

5-(((3-(1-Benzylpiperidin-4-yl)propyl)(prop-2-ynyl)amino)methyl)quinolin-8-ol (**7**). To a cooled mixture of amine **20** (189 mg, 0.70 mmol) and Et₃N (0.51 ml, 2.5 mmol) in CH₂Cl₂ (15 mL) at 0 °C, chloride **9** (186 mg, 0.73 mmol) was added. After being stirred at rt overnight, the reaction was quenched with aqueous 5% NaHCO₃. The product was extracted with CH₂Cl₂, and extracts were dried over Mg₂SO₄ and then concentrated in vacuo. The crude products were purified by column chromatography (SiO₂, hexane/AcOEt by increasing the gradient from 5:1 to 1:1 v/v) to give compound 7 (162 mg, 54.3%) as a yellow oil: IR (film) ν 3381, 3293, 2911, 2801, 1738, 1580, 1505, 1476, 1425, 1373, 1289, 1238, 1198, 1047 cm⁻¹; ¹H NMR (300 MHz, CDCl₃) δ 0.99-1.33 (m, 5 H), 1.42-1.66 (m, 4 H), 1.86 (t, *J*= 11.3 Hz, 2 H), 2.29 (t, *J*= 2.1 Hz, 1 H), 2.56 (t, *J*= 7.1 Hz, 2 H), 2.86 (d, *J*= 11.4 Hz, 2 H), 3.26 (d, *J*= 2.2 Hz, 2 H), 3.48 (s, 2 H), 3.93 (s, 2 H), 7.09 (d, *J*= 7.6 Hz, 1 H), 7.20-7.37 (m, 5 H), 7.37-7.49 (m, 2 H), 8.65 (dd, *J*= 8.5, 1.3 Hz, 1 H), 8.77 (dd, *J*= 4.1, 1.3 Hz, 1 H); ¹³C NMR (100 MHz, CDCl₃) δ 24.4, 32.2, 34.0, 35.3, 40.9, 53.2, 53.8, 55.8, 63.4, 73.4, 78.4, 108.9, 121.4, 124.9, 127.0, 127.9, 128.2, 129.2, 129.3, 134.1, 138.7, 147.6, 152.0. Anal. Calcd for C₂₈H₃₃N₃O: C, 78.65; H, 7.78; N, 9.98. Found; C, 76.90; H, 8.03; N 9.83.

4-(1-Benzylpiperidin-4-yl)-2-((8-hydroxyquinolin-5-yl)methyl)(prop-2-yny)amino)butanenitrile (**6**). To a mixture of amine **21** (89 mg, 0.3 mmol) and Et₃N (0.09 mL, 0.7 mmol) in CH₂Cl₂ (6 mL) at 0 °C, chloride **9** (77 mg, 0.3 mmol) was added. After being stirred at rt overnight, the reaction was quenched with aqueous 5% NaHCO₃ and the product was extracted with CH₂Cl₂.The extracts were dried Mg₂SO₄, and concentrated in vacuo. The crude products were purified by column chromatography (SiO₂, hexane/AcOEtby increasing the gradient from 5:1 to 1:1 v/v), to give compound **6** (89 mg, 65.4%) as a yellow oil: IR (film) ν 3295, 2931, 2812, 1578, 1505, 1476, 1371, 1271, 1203, 1196, 1148, 1123, 1072, 1028 cm-¹; ¹H NMR (400 MHz, CDCl₃) δ 0.90 (m, 1 H), 1.03-1.35 (m, 4 H), 1.45 (d, 1 H), 1.73-1.76 (m, 4 H), 2.42 (s, 1H), 2.80 (t, *J*= 14.40 Hz, 2H), 3.29 (m, 1 H), 3.45 (s, 3 H), 3.58 (t, *J*= 8.00 Hz, 1 H), 3.75 (d, *J*= 12.8 Hz, 1 H), 4.58 (d, *J*= 13.2 Hz, 1H), 7.11 (t, *J*= 7.60 Hz, 1 H), 7.29-7.46 (m, 7 H), 8.61-8.64 (m, 1 H), 8.78-8.80 (m, 1 H); ¹³C NMR (100 MHz, CDCl₃) δ 28.1, 31.5, 31.9, 32.0, 34.4, 39.8, 52.3, 53.4, 53.5, 53.7, 63.3, 74.2, 78.6, 108.9, 117.2, 121.8, 122.5, 127.1, 127.6, 128.2, 129.3, 129.9, 133.7, 138.8, 147.9, 152.6. HRMS. Calcd for C₂₉H₃₂N₄O (MH+): 453.2654. Found: 452.2526 (MH+).

### BIOLOGICAL ASSAYS

### Example 7. Metal binding assay

It is known that 8-quinolinol is a strong chelator for biometals such iron and copper. This is an important precondition for the antioxidative-type drugs because it is the excessive iron stores and iron-mediated generation of free radicals in the brain that are thought to be associated with neurodegenerative diseases. Therefore, only chelators with a higher selectivity for iron over copper are expected to chelate iron instead of copper and have potential neuroprotective effects. In order to discuss possible correlation between chelating properties of 8-quinolinol and its derivatives with their antioxidative ability, and the correlation between its derivative and the best established iron chelating drug, desferal, with antioxidative properties, a reliable measurement of the stability constants of the newly synthesized compounds is necessary. A spectrophotometric method is used for measurement of the copper/iron-complexes stability constants of the compounds.

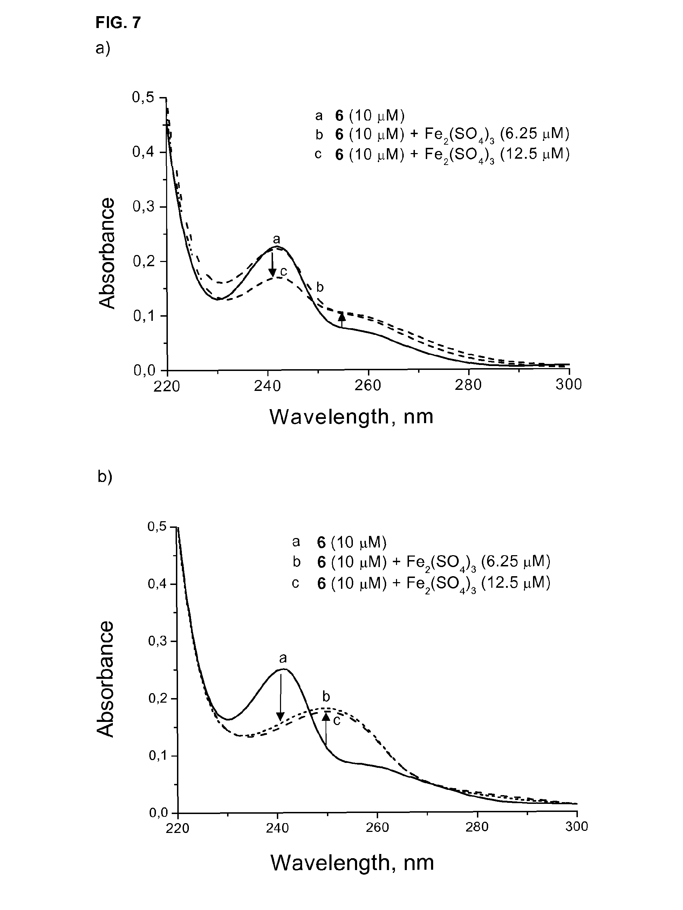

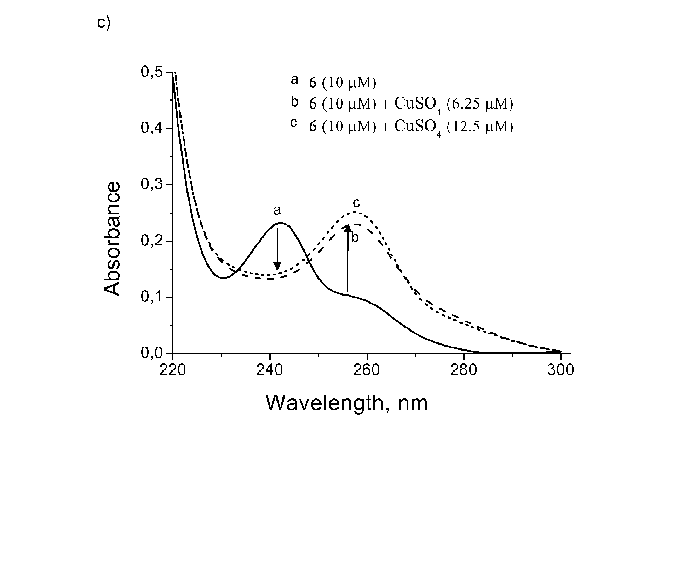

The metal binding assay was performed by the spectrophotometric detection of the formation of Fe(III)- and Cu(II)-**6** complex. Compound **6** absorbance spectrum at 10µM was assessed from 220 nm to 300 nm for both biometals revealing the appearance of two absorption maximas around 240nm and 257nm [Fig. 7]. The formation of Cu(II)-**6** complex was easily determined just few minutes after the addition of CuSO₄ solutions. The 240 nm-band completely dissapeared while a new one appeared at 275 nm in both Cu(II) concentrations performed. As far as iron chelation is concerned, incubation time became a limiting factor to detect some absorbance shift. Few minutes after the addition of Fe₂(SO₄)₃ no significative changes in the spectrum were observed. A new detection was performed after overnight incubation revealing a bathocromic shift of the 240nm-band to the appearance of another one at 250nm. In conclusion, iron chelation process by **6** is much slower than copper at same time and concentration conditions.

### Example 8. MAO inhibitory activity

### Preparation of rat brain MAO

Rats were decapitated and brains weighted and quickly taken into ice-cold Tris 10 nM pH 7.2 and 0.25M saccharose buffer. All subsequent procedures were performed at 4°C approximately. The brains were homogenized in 0.25 M Tris-saccharose buffer in a Teflon glass homogenizer followed by the addition of the Tris-buffer to a final concentration of 10% homogenate. The homogenates were filtered with a surgical gauze and centrifuged twice at 600 g for 10 min. The pellet was ressuspended in 0.25M Tris-saccharose buffer and centrifuged twice at 10000g for 10 min. Finally, a protein stock at 10 mg/ml ressuspended in 0.1 M potassium phosphate pH 7.4 buffer was prepared and kept frozen at -20°C for later assaying of MAO. Protein concentration was determined with Bradford reagent at [λ]=595 nm, using albumin from bovine serum (BSA) as a standard.

### Inhibition of MAO-A and MAO-B

MAO-A and MAO-B activities were determined in rat brain homogenate *in vitro* incubation with varying concentrations of the test compounds following the method of Fowler and Tipton (C. J. Fowler, K. F. Tipton, Biochem. Pharmacol. 1981, 30, 3329-3332).

The in vitro inhibitory activity of the compounds was tested against the two rat brain MAO isoforms. In order to test their multipotent profile, DHP 2-7 dilutions were added to potassium phosphate buffer pH 7.4 and incubated with a suitable dilution of enzyme preparation (50-100mg of total protein) for 30 min at 37 °C before the addition of ¹⁴C-labelled MAO-A and MAO-B substrates; 100µM 5-hydroxytryptamine (5-HT) and 20 µM phenylethylamine (PEA) respectively. Enzymatic reactions, 20 min for MAO-A and 4 minutes for MAO-B, were stopped with 2M citric acid.

Metabolites were extracted and determined by liquid-scintillation counting in cpm units and expressed in enzymatic activity units (pmol/min·mg prot.).

Clorgyline and deprenyl inhibitory profiles were also performed as a MAO-A and MAO-B specific inhibitors, respectively.

**Table 1. Inhibition [IC50(µM)] of rat liver monoamine oxidase (MAO-A and MAO-B), and bovine lung SSAO by compounds of the invention.**

| **Compound** | **bovineSSAO** | **ratMAO-A** | **ratMAO-B** | **MAO-B/ MAO-A** |
|---|---|---|---|---|
| **M-30^{(a)}** | - | 0.037 ± 0.02 | 0.057 ± 0.02 | 1.5 |
| **Donepezil** 1 | - | 850 ± 13 | 15 ± 2.2 | 0.020 |
| **2** | 17.98 ± 5.0 | 22.07 ± 0.7 | 39.48 ± 1.4 | 1.7 |
| **3** | ≥100 | 85.36 ± 3.7 | 19.41 ± 3.2 | 0.22 |
| **4** | 2.79 ± 0.7 | 9.67 ± 1.5 | 12.36 ± 2.5 | 1.2 |
| **5** | 39.71 ± 16.0 | ≥ 100 | 50.08 ± 0.5 | ≥ 0.5 |
| **6** | 2.01 ± 0.5 | 6.16 ± 0.7 | 10.21 ± 0.9 | 1.7 |
| **7** | 61.08 ± 21.4 | ≥ 100 | 34.54 ± 3.5 | ≥ 0.34 |

| | | | | |
|---|---|---|---|---|
| ^{(a)} H. Zheng, M. B. H. Youdim, M. Fridkin, ACS Chem. Biol. 2010, 5, 603-610. | | | | |

Values are expressed as mean ± standard error of the mean of at least three different experiments in quadruplicate.

As shown in Table 1, most of compound derivatives were low MAO inhibitors, compounds **4** and **6** show moderate, almost equipotent inhibitory activity on both enzymes, the most potent being compound **6** [ratMAO-A (IC₅₀= 6.16 ± 0.7 µM; ratMAO-B (IC₅₀= 10.21 ± 0.9 µM)]. Comparing with the reference compound **M-30,** the most potent inhibitor compound **6** is 166- and 179-fold less active for the inhibition of MAO-A and MAO-B, respectively. However, if compared to donepezil **1,** inhibitor **6** is 138-fold more active, while remains equipotent for MAO-A and MAO-B, respectively. As before, the same structure-activity relationship applies: for the same value of n, the α-aminonitriles are more potent, with the exception of compound **2** for MAO-B, than the amines for both enzymes.

Regarding the inhibition of SSAO, we have observed the same biological trends (see Table 1), the most potent being compound **6** [bovineSSAO (IC₅₀= 2.01 ± 0.5 µM)], in the same range as compound **4.** This result is particularly interesting, as it is well known that whereas MAO catalyses the oxidative deamination of primary, secondary and tertiary amines, SSAO activity appears to be restricted to primary amines.

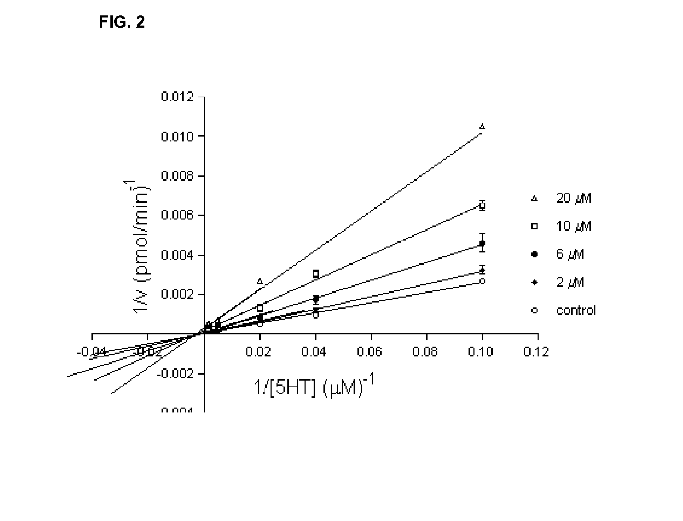

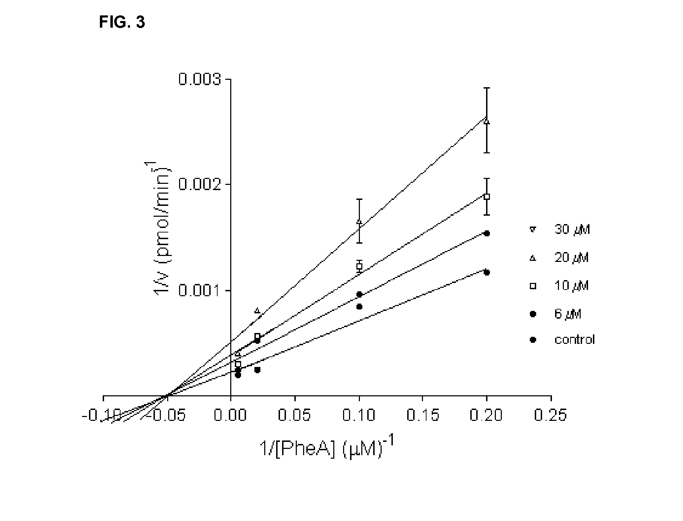

In order to determine the type of the MAO inhibition mechanism of the compounds of the invention, a kinetic study was carried out with compound **6.** A non-competitive inhibition was established from the analysis of Lineweaver-Burk reciprocal plots on both enzymes [Figure 2 (MAO-A); Figure 3 (MAO-B)]. *K*ᵢ values of 8.41 ± 1.32 µM and 10.81 ± 2.43 µM, respectively, were estimated from the slopes of double reciprocal plots versus compound **6** concentrations.

Next, the reversibility of the inhibition of compound **6** has been addressed. We observed that compound **6** reversibly inhibited both MAO-A and MAO-B as inhibitions are significantly reverted after three consecutive centrifugations and washings with buffer (Figure 4). Simultaneously, the reversibility of *clorgyline,* as an irreversible inhibitor of MAO-A and *I-deprenyl,* as an irreversible inhibitor of MAO-B, were also performed as reference compounds.

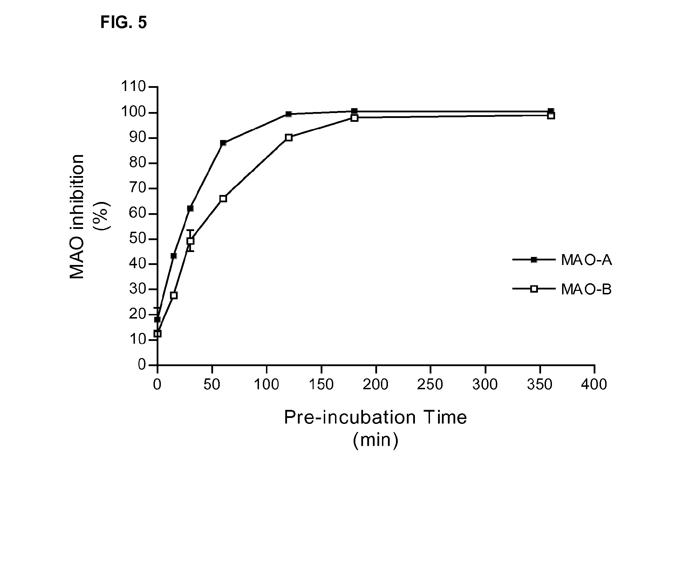

In order to corroborate the reversibility results obtained, time-dependence inhibition assessments for long pre-incubation times (0-360 min) were also carried out over both MAO-A and MAO-B activities in presence of compound **6** (Figure 5). In this case concentrations of compound **6** used were 20 µM for both enzymes.

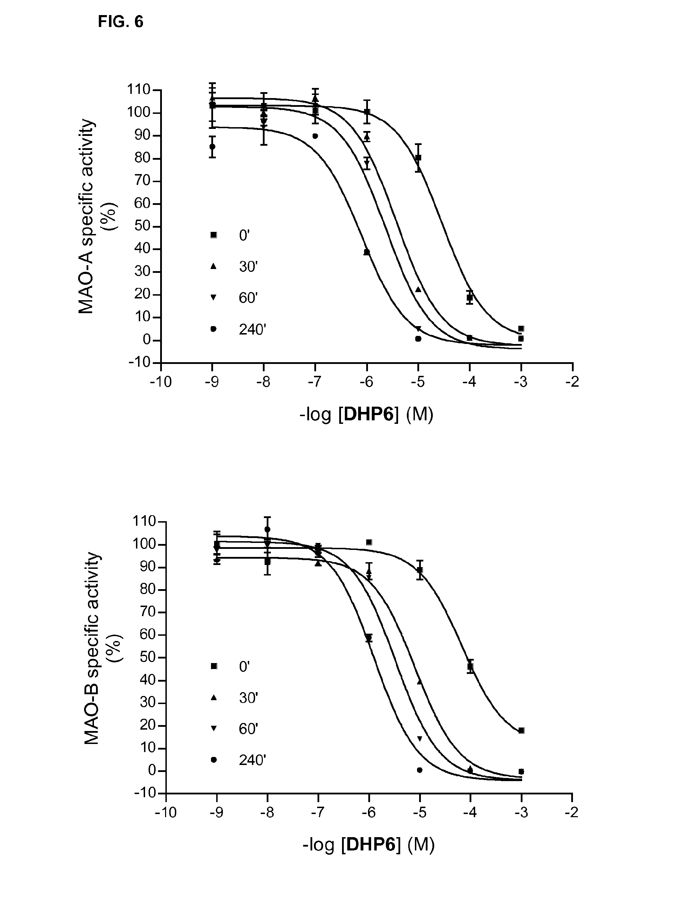

A complete activity inhibition was not reached before 180 minutes of pre-incubation period for both MAO-A and MAO-B, revealing therefore an irreversible behaviour. Independent inhibition assays were also performed at different pre-incubation periods for both two MAO isoforms (Figure 6). IC₅₀ values (Table 2) showed a progressive increase of the compound potency as the pre-incubation times were longer, verifying previous results.

**Table 2. Inhibition [IC₅₀(µM)] of MAO-A and MAO-B from rat liver by compound 6 at different pre-incubation times (0-240 min).**

| **IC₅₀ (µM) by pre-incubation time (min)** | | | | |
|---|---|---|---|---|
| | **0** | **30** | **60** | **240** |
| **MAO-A** | 29.1 | 5.8 | 2.3 | 0.7 |
| **MAO-B** | 61.7 | 9.3 | 3.3 | 1.2 |

### Example 9. ChE inhibitory activity

The in vitro activity of the new compounds against *electrophorus electricus* acetylcholinesterase (eeAChE) and *equine serum* butyrylcholinesterase (eqBuChE) activities was determined using Ellman's method (G. L. Ellman, et al., Biochem. Pharmacol. 1961, 7, 88-95) with donepezil and M30 as reference compounds (Table 3).

Diluted solutions of DHP 2-7 derivatives were prepared and added to a 333 µM of 5,5'-dithiobis 2-nitrobenzoic acid (DTNB), 0.1 M phosphate buffer with 0.02% BSA pH8 and enzyme solutions: eeAchE at 0.02 U/ml and eqBuChE at 0.017 U/ml. Solutions were incubated for 20 minutes at 37°C prior the substrate addition. Acetylthiocholine iodide (450 µM final concentration) and S-butyrylthiocholine iodide (300µM final concentration) were used as substrates for eeAChE and eqBuChE respectively. Once added, reaction times used were 5 minutes for *Ee*AChE and 30 minutes for eqBuChE.

Next, absorbance was determined at 405nm and expressed in activity percentage in order to determine reliable IC₅₀ values for both cholinesterases.

**Table3. Inhibition [IC₅₀(µM)] of AChE from electrophorus electricus (EeAChE), equine serum butyrylcholinesterase (eqBuChE) by compounds of the invention.**

| **Compound** | **EeAChE** | **eqBuChE** | **BuChE/ AChE** |
|---|---|---|---|
| **M-30^{(a)}** | 113.86 ± 14.05 | ≥ 100 | - |
| **Donepezil 1** | 0.0067 ± 0.0004 | 7.4 ± 0.1 | 1100 |
| **2** | 14.53 ± 3.7 | 13.5 ± 1.8 | 0.92 |
| **3** | 17.03 ± 4.7 | 12.8 ± 1.4 | 0.75 |
| **4** | 5.53 ± 0.5 | 6.06 ± 0.6 | 1.1 |
| **5** | 5.01 ± 0.8 | 14.47 ± 3.6 | 2.8 |
| **6** | 1.77 ± 0.1 | 1.63 ± 0.25 | 0.92 |
| **7** | 2.67 ± 0.4 | 4.52 ± 0.3 | 1.6 |

| | | | |
|---|---|---|---|
| ^{(a)}H. Zheng, M. B. H. Youdim, M. Fridkin, ACS Chem. Biol. 2010, 5, 603-610. | | | |

Values are expressed as mean ± standard error of the mean of at least three different experiments in quadruplicate.

Compared to donepezil **1,** compound **6** is significantly less potent for the inhibition of EeAChE, but more active (4.5-fold) for eqBuChE inhibition. Comparing with M-30, compound **6** is 64-fold more active for the inhibition of AChE, and more efficient for the inhibition of BuChE, as M-30 is inactive on this enzyme.

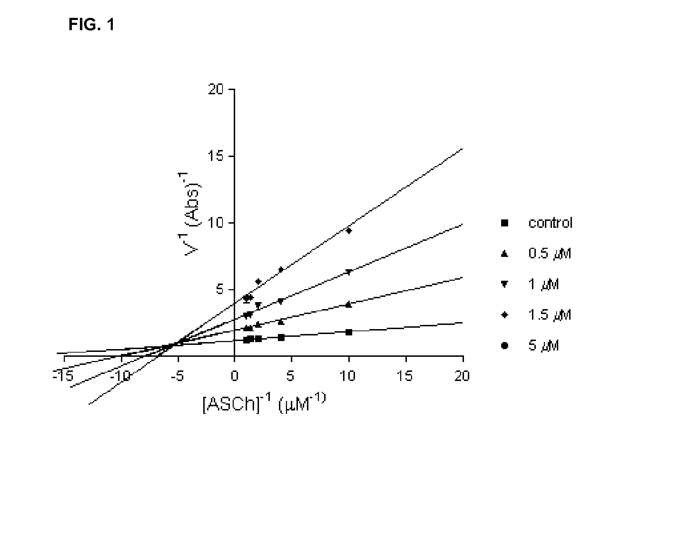

To determine the type of the *E*eAChE inhibition mechanism of these compounds, a kinetic study was carried out on inhibitor **6.** The type of inhibition was established from the analysis of Lineweaver-Burk reciprocal plots (Figure 1) showing both increasing slopes (lower *V*ₘₐₓ) and intercepts (higher *K*ₘ) with higher inhibitory concentration. This suggests a mixed-type inhibition.^{[36]} The graphical analysis of steady-state inhibition data for compound **6** is shown in Figure 1. A *K*ᵢ value of 1.21 ± 0.25 µM was estimated from the slopes of double reciprocal plots versus compound **6** concentrations.

Compounds **2-7** were readily prepared in good yields, in short synthetic sequences, from easily available precursors. The biological evaluation of molecules **2-7** showed that these compound hybrids are moderate, in the micromolar range, non-selective ChE and MAO inhibitors. Particularly interesting resulted compound **6,** a reversible, non-competitive MAO-A/ MAO-B, and a mixed-type EeAChE inhibitor. Compared to related **M-30A,** the demethylated derivative of M-30, or **M-30B** ["5,5'-((prop-2-yn-1-ylazanediyl)bis(methylene))bis(quinolin-8-ol)"] [H. Zheng, S. Gal, L. M. Weiner, O. Bar-Am, A. Warshawsky, M. Fridkin, M. B. H. Youdim, J. Neurochem. 2005, 95, 68-78.] compound **6** shows a quite similar MAO inhibition profile.

### REFERENCES CITED IN THE APPLICATION

WO2004/411 (Yeda Res. Dev. Co.)
WO2010/86860 (Yeda Res. Dev. Co.)
C. J. Fowler, et al., Biochem. Pharmacol. 1981, 30, 3329-3332
H. Zheng, et al., ACS Chem. Biol. 2010, 5, 603-610
G. L. Ellman, et al., Biochem. Pharmacol. 1961, 7, 88-95
H. Zheng, et al., J. Neurochem. 2005, 95, 68-78.

## Claims

1. A compound of formula (I) 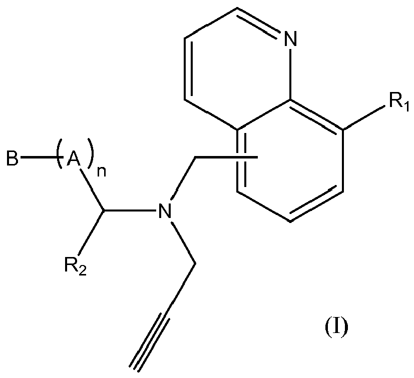 or a salt thereof, or any of its stereoisomeric forms or a mixture thereof, wherein
R₁ and R₂, same or different, are each independently selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; halo-(C₁-C₆)alkyl; linear or branched (C₂-C₆)alkenyl; F; Cl; Br; 1; -NO₂; -NH₂; -CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl, - SON((C₁-C₄)alkyl)₂; -OCONH₂; -OCONH(C₁-C₄)alkyl; -OCON((C₁-C₄)alkyl)₂; - C=N(C₁-C₄)alkyl; -NHC(O)(C₁-C₄)alkyl; -N(C₁-C₄)alkylC(O)(C₁-C₄)alkyl; - N(C₁-C₄)alkylC(O)H; -N=CH₂; -N=CH(C₁-C₄)alkyl; -N=C((C₁-C₄)alkyl)₂; phenylcarbonyl; phenylcarbonyl, the phenyl moiety being substituted by at least a radical selected from the group consisting of halo(C₁-C₆)-alkyl, OH, halogen, (C₁-C₆)-alkyl, (C₁-C₆)-alkoxy, and CN; (C₁-C₃)-alkylphenylcarbonyl; (C₁-C₃)-alkylphenylcarbonyl being the phenyl moiety substituted by at least a radical selected from the group consisting of halo(C₁-C₆)-alkyl, OH, halogen, (C₁-C₆)-alkyl, (C₁-C₆)-alkoxy, and CN; phenyl; phenyl substituted by at least a radical selected from the group consisting of halo(C₁-C₆)-alkyl, OH, halogen, (C₁-C₆)-alkyl, (C₁-C₆)-alkoxy, and CN; (C₁-C₃)-alkylphenyl; (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN;
n is an integer from 0 to 1;
A is selected from the group consisting of
(i) -(CH₂)ₙ₁-X-(CH₂)ₙ₂- wherein n1 and n2 are an integer each independently selected from 0 to 6;
(ii) -X-(CH₂)ₙ₃-Y- wherein n3 is an integer from 1 to 6;
(iii) a bivalent radical selected from the group consisting of 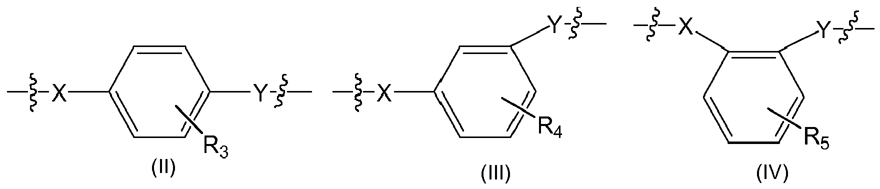
wherein R₃, R₄ and R₅ are selected from H, OH, halogen, CN, (C₁-C₄)alkyl, hydroxy-(C₁-C₄)alkyl; halo(C₁-C₆)-alkyl, and (C₁-C₆)alkoxy;
wherein X and Y are each independently selected from O, N and CH₂;
and wherein the wavy line indicates the position through which the A moiety binds, being the X atom bound to the B moiety and the Y atom bound to the backbone of the compound of formula I;
B is selected from the group consisting of 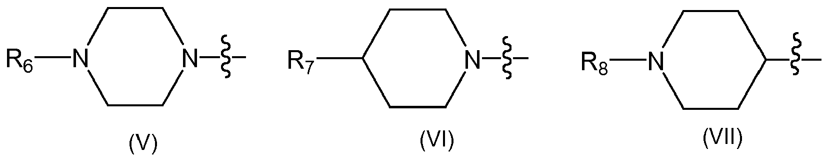 wherein the wavy line indicates the position through which the B moiety binds to the X atom of the A moiety, and wherein R₆, R₇ and R₈ are selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; halo-(C₁-C₆)alkyl; linear or branched (C₂-C₆)alkenyl; F; Cl; Br; I; -NO₂; -NH₂; -CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)N((C₁-C₄)alkl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C ₄)alkyl; -SO₂-(C₁-C₄)alkyl; -SO₂NH₂; -SONH(C₁-C₄)alkyl; - SON((C₁-C₄)alkyl)₂; -OCONH₂; -OCONH(C₁-C₄)alkyl; -OCON((C₁-C₄)alkyl)₂; - C=N(C₁-C₄)alkyl; -NHC(O)(C₁-C₄)alkyl; -N(C₁-C₄)alkylC(O)(C₁-C₄)alkyl; - N(C₁-C₄)alkylC(O)H; -N=CH₂; -N=CH(C₁-C₄)alkyl; -N=C((C₁-C₄)alkyl)₂; phenylcarbonyl; phenylcarbonyl, the phenyl moiety being substituted by at least a radical selected from the group consisting of halo(C₁-C₆)-alkyl, OH, halogen, (C₁-C₆)alkyl, (C₁-C₆)alkoxy, and CN; (C₁-C₃)-alkylphenylcarbonyl; (C₁-C₃)-alkylphenylcarbonyl being the phenyl moiety substituted by at least a radical selected from the group consisting of halo(C₁-C₆)-alkyl, OH, halogen, (C₁-C₆)alkyl, (C₁-C₆)alkoxy, and CN; phenyl; phenyl substituted by at least a radical selected from the group consisting of halo(C₁-C₆)-alkyl, OH, halogen, (C₁-C₆)alkyl, (C₁-C₆)alkoxy, and CN; (C₁-C₃)-alkylphenyl; (C₁-C₃)-alkylphenyl, being the phenyl moiety substituted by at least a radical selected from the group consisting of halo(C₁-C₆)-alkyl, OH, halogen, (C₁-C₆)alkyl, (C₁-C₆)alkoxy, and CN; and a C-radical of one of the known carbocyclic or heterocyclic ring systems with 1-4 rings, wherein each one of the rings forming said ring system
has 5-7 members, each member independently selected from C, N, O, S, CH, CH₂, NH,
is saturated, partially unsaturated or aromatic, and
is isolated, partially or totally fused;
being each ring forming part of the ring system optionally substituted by at least one radical selected from the group consisting of H;
OH; -CH₂-NH-CH₂CCH; -NH-
CH₂CCH; -CH₂-N(C₁-C₄)alkylCH₂CCH; -N(C₁-C₄)alkylCH₂CCH;linear or branched (C₁-C₆)alkyl; hydroxy-(C₁-C₆)alkyl; halo-(C₁-C₆)alkyl; linear or branched (C₂-C₆)alkenyl; F; Cl; Br; I; -NO₂; -NH₂; -CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-NH(C₁-C₄)alkyl; -SH; -S(C₁-C₄)alkyl; -SO-(C₁-C ₄)alkyl; -SO₂-(C₁-C₄)alkyl; -SO₂NH₂; -SONH(C₁-C₄)alkyl; - SON((C₁-C₄)alkyl)₂; -OCONH₂; -OCONH(C₁-C₄)alkyl; -OCON((C₁-C₄)alkyl)₂; - C=N(C₁-C₄)alkyl; -NHC(O)(C₁-C₄)alkyl; -N(C₁-C₄)alkylC(O)(C₁-C₄)alkyl; - N(C₁-C₄)alkylC(O)H; -N=CH₂; -N=CH(C₁-C₄)alkyl; -N=C((C₁-C₄)alkyl)₂;
with the proviso that wherein the A moiety is -X-(CH₂)ₙ"-Y-, or a radical of formula (II), (III) or (IV) and X is N or O, then B is a radical of formula (VII).

2. The compound according to claim 1 wherein B is a radical of formula (VII);
wherein R₈ is a radical as defined in claim 1.

3. The compound according to any of claims 1-2 wherein R₈ is selected from the group consisting of H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)NHCH₃; -C(O)N(CH₃)₂; -NH₂; -NHCH₃; -N(CH₃)₂; - CH₂NH₂; -CH₂CH₂NH₂; -CH₂NHCH₃; -CH₂N(CH₃)₂; -CH₂CH₂N(CH₃)₂; - CH₂N(CH₂CH₃)₂; -CH₂CH₂N(CH₂CH₃)₂; -SH; -SCH₃; -SCH₂CH₃; -SO-CH₃; - SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; -C=NH; -C=NCH₃; - C=NCH₂CH₃; -NCH₃C(O)CH₃; -NCH₃C(O)CH₂CH₃; -N=CH₂; -N=C(CH₃)₂; - N=C(CH₂CH₃)₂; phenylcarbonyl; phenylcarbonyl, the phenyl moiety being substituted by at least a radical selected from the group consisting of CF₃, F, Cl, Br, I, CN, OH, -CH₃, -CH₂CH₃, -OCH₃, and -OCH₂CH₃; - (C₁-C₃)-alkylphenylcarbonyl, (C₁-C₃)-alkylphenylcarbonyl being the phenyl moiety substituted by at least a radical selected from the group consisting of CF₃, F, Cl, Br, I, CN, OH, -CH₃, -CH₂CH₃, -OCH₃, and -OCH₂CH₃; phenyl; phenyl substituted by at least a radical selected from the group consisting of CF₃, F, Cl, Br, I, CN, OH, -CH₃, -CH₂CH₃, -OCH₃, and -OCH₂CH₃; (C₁-C₃)-alkylphenyl; (C₁-C₃)-alkylphenyl, being the phenyl moiety substituted by at least a radical selected from the group consisting of CF₃, F, Cl, Br, I, CN, OH, -CH₃, -CH₂CH₃, -OCH₃, and -OCH₂CH₃.

4. The compound according to any of claims 1-3, wherein n is an integer from 0 to 1, and the B-A moiety is selected from the group consisting of B-(CH₂)ₙ₁-O-(CH₂)ₙ₂-; B-(CH₂)ₙ₁-NH-(CH₂)ₙ₂-; B-(CH₂)ₙ₁-CH₂-; B-(CH₂)ₙ₁-O-; B-(CH₂)ₙ₁-NH-; B-O-(CH₂)ₙ₂-; B-NH-(CH₂)ₙ₂-; B-; B-O-; B-NH-; B-O-(CH₂)ₙ₃-O-; B-O-(CH₂)ₙ₃-NH-; B-NH-(CH₂)ₙ₃-NH-; and B-NH-(CH₂)ₙ₃-O-;
being B as defined in claim 1; and n1, n2 and n3 are an integer each independently selected from 1 to 4.

5. The compound according to any of claims 1-3, wherein n is 1, A moiety is selected from a bivalent radical selected from the group consisting of 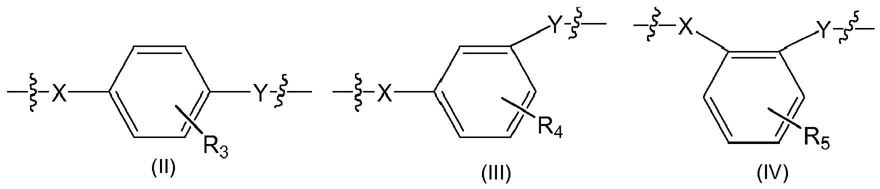 wherein R₃, R₄ and R₅ are selected from H, OH, halogen, CN, (C₁-C₄)alkyl, halo(C₁-C₆)-alkyl, and (C₁-C₆)alkoxy;
wherein X and Y are each independently selected from O, N and CH₂;
and wherein the wavy line indicates the position through which the A moiety binds, being the X atom bound to the B moiety and the Y atom bound to the backbone of the compound of formula (I).

6. The compound according to any of claims 1-5, wherein R₁ and R₂, same or different, are each independently selected from the group consisting of H; OH; -CH₂-NH-CH₂CCH; -NH-CH₂CCH; -CH₂-N(C₁-C₄)alkylCH₂CCH; - N(C₁-C₄)alkylCH₂CCH; linear or branched (C₁-C₆)alkyl; hydroxy-(C₁-C₆)alkyl; halo-(C₁-C₆)alkyl; -NH₂; -CN; -C(O)(C₁-C₄)alkyl; -COO(C₁-C₄)alkyl; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-
NH(C₁-C₄)alkyl; -SH; -S(C₁-C₄)alkyl; -SO-(C₁-C₄)alkyl; -SO₂-(C₁-C₄)alkyl; -SO₂ NH₂; -SONH(C₁-C₄)alkyl; -
SON((C₁-C₄)alkyl)₂; -OCONH₂; -OCONH(C₁-C₄)alkyl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN.

7. The compound according to any of claims 1-6, wherein R₁ is selected from the group consisting of OH; -NH₂; -CN; -C(O)(C₁-C₄)alkyl; -COO(C₁-C₄)alkyl; - OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-
NH(C₁-C₄)alkyl; -SH; -S(C₁-C₄)alkyl; -SO-(C₁-C₄)alkyl; -SO₂-(C₁-C₄)alkyl; -SO₂ NH₂; -SONH(C₁-C₄)alkyl; -SON((C₁-C₄)alkyl)₂; -OCONH₂; -OCONH(C₁-C₄)alk yl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN.

8. The compound according to any of claims 1-7, which is that of formula (Ia) 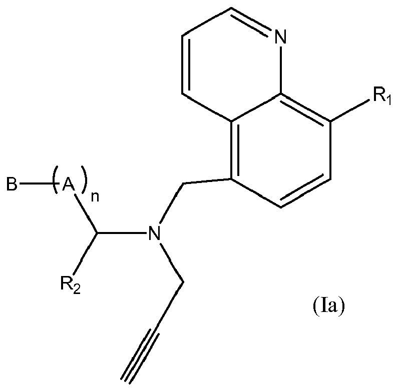 wherein
R₁ is selected from OH; -NH₂; -
CN; -C(O)(C₁-C₄)alkyl; -COO(C₁-C₄)alkyl; -
OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-
NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl; -SON((C₁-C₄)alkyl)₂; -OCONH₂; -OCONH(C₁-C₄)al kyl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN;
R₂ is selected from H; OH; -CH₂-NH-CH₂CCH; -NH-CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; halo-(C₁-C₆)alkyl; -NH₂; - CN; -C(O)(C₁-C₄)alkyl; -COO(C₁-C₄)alkyl; -
OC(O)(C₁-C₄)alkyl; -C(O)N((C₁-C₄)alkyl)₂; -NH(C₁-C₄)alkyl; - N((C₁-C₄)alkyl)₂; -(C₁-C₄)alkyl-
NH(C₁-C₄)alkyl; -SH; -S(C₁-C₄)alkyl; -SO-(C₁-C₄)alkyl; -SO₂-(C₁-C₄)alkyl; -SO ₂NH₂; -SONH(C₁-C₄)alkyl, -
SON((C₁-C₄)alkyl)₂; -OCONH₂; -OCONH(C₁-C₄)alkyl; -OCON((C₁-C₄)alkyl)₂; and (C₁-C₃)-alkylphenyl, being the phenyl moiety substituted by at least a radical selected from the group consisting of halo(C₁-C₆)-alkyl, OH, halogen, (C₁-C₆)-alkyl, (C₁-C₆)-alkoxy, and CN;
B is a radical of formula VII, wherein R₈ is selected from the group consisting of H; OH; -CH₂-NH-CH₂CCH; -NH-
CH₂CCH; -CH₂-N(C₁-C₄)alkylCH₂CCH; -N(C₁-C₄)alkylCH₂CCH; linear or branched (C₁-C₆)alkyl; hydroxy-(C₁-C₆)alkyl; CF₃; CN; -O(C₁-C₄)alkyl; (C₁-C₄)alkyl-O-(C₁-C₄)alkyl; - C(O)H; -C(O)(C₁-C₄)alkyl; -COOH; -COO(C₁-C₄)alkyl; -OC(O)H; - OC(O)(C₁-C₄)alkyl; -C(O)NHCH₃; -C(O)N(CH₃)₂; -NH₂; -NHCH₃; -N(CH₃)₂; - CH₂NH₂; -CH₂CH₂NH₂; -CH₂NHCH₃; -CH₂N(CH₃)₂; -CH₂CH₂N(CH₃)₂; - CH₂N(CH₂CH₃)₂; -CH₂CH₂N(CH₂CH₃)₂; -SH; -SCH₃; -SCH₂CH₃; -SO-CH₃; - SO-CH₂CH₃; -SO₂-CH₃; -SO₂-CH₂CH₃; -SO₂N(CH₃)₂; -SO₂NHCH₃; - NO₂; -OCON(CH₃)₂; -OCONHCH₃; -OCON(CH₂CH₃)₂; -C=NH; -C=NCH₃; - C=NCH₂CH₃; -NCH₃C(O)CH₃; -NCH₃C(O)CH₂CH₃; -N=CH₂; -N=C(CH₃)₂; - N=C(CH₂CH₃)₂; phenylcarbonyl; phenylcarbonyl, the phenyl moiety being substituted by at least a radical selected from the group consisting of CF₃, F, Cl, Br, I, CN, OH, -CH₃, -CH₂CH₃, -OCH₃, and -OCH₂CH₃; - (C₁-C₃)-alkylphenylcarbonyl, (C₁-C₃)-alkylphenylcarbonyl being the phenyl moiety substituted by at least a radical selected from the group consisting of CF₃, F, Cl, Br, I, CN, OH, -CH₃, -CH₂CH₃, -OCH₃, and -OCH₂CH₃; phenyl;
phenyl substituted by at least a radical selected from the group consisting of CF₃, F, Cl, Br, I, CN, OH, -CH₃, -CH₂CH₃, -OCH₃, and -OCH₂CH₃; (C₁-C₃)-alkylphenyl; (C₁-C₃)-alkylphenyl, being the phenyl moiety substituted by at least a radical selected from the group consisting of CF₃, F, Cl, Br, I, CN, OH, -CH₃, -CH₂CH₃, -OCH₃, and -OCH₂CH₃;
wherein n is an integer from 0 to 1,
and the B-A moiety is selected from the group consisting of B-(CH₂)ₙ₁-O-(CH₂)ₙ₂-; B-(CH₂)ₙ₁-NH-(CH₂)ₙ₂-; B-(CH₂)ₙ₁-CH₂-; B-(CH₂)ₙ₁-O-; B-(CH₂)ₙ₁-NH-; B-O-(CH₂)ₙ₂-; B-NH-(CH₂)ₙ₂-; B-; B-O-; B-NH-; B-O-(CH₂)n3-O-; B-O-(CH₂)ₙ₃-NH-; B-NH-(CH₂)ₙ₃-NH-; and B-NH-(CH₂)ₙ₃-O-;
being B as defined above; and n1, n2 and n3 are an integer each independently selected from 1 to 4.

9. The compound according to any of claims 1-8, which is that of formula (Ia) wherein
R₁ is selected from OH; -NH₂; -CN; -C(O)CH₃; -COOCH₃;
and -OC(O)CH₃;
R₂ is selected from H; OH; and -CN;
B is a radical of formula (VII), wherein R₈ is selected from the group consisting of H; OH; methyl; ethyl; -CH₂OH; -NH₂; -
CN; -C(O)CH₃; -COOCH₃; -OC(O)CH₃; -OCONH₂; phenyl; and benzyl;
wherein n is an integer from 0 to 1,
the B-A moiety is selected from the group consisting of B-(CH₂)ₙ₁-0-(CH₂)ₙ₂-; B-(CH₂)ₙ₁-NH-(CH₂)ₙ₂-; B-(CH₂)ₙ₁-CH₂-; B-(CH₂)n1-O-; B-(CH₂)ₙ₁-NH-; B-O-(CH₂)ₙ₂-; B-NH-(CH₂)ₙ₂-; B-; B-O-; B-NH-; B-O-(CH₂)ₙ₃-O-; B-O-(CH₂)ₙ₃-NH-; B-NH-(CH₂)ₙ₃-NH-; and B-NH-(CH₂)ₙ₃-O-;
and n1, n2 and n3 are an integer each independently selected from 1 to 2.

10. The compound according to any of claims 1-9, which is selected from
2-(1-Benzylpiperidin-4-yl)-2-(((8-hydroxyquinolin-5-yl)methyl)(prop-2-ynyl)amino)acetonitrile (2);
5-((((1-Benzylpiperidin-4-yl)methyl)(prop-2-ynyl)amino)methyl)quinolin-8-ol (**3**);
3-(1-Benzylpiperidin-4-yl)-2-(((8-hydroxyquinolin-5-yl)methyl)(prop-2-ynyl)amino)propanenitrile (**4**);
5-(((2-(1-Benzylpiperidin-4-yl)ethyl)(prop-2-ynyl)amino)methyl)quinolin-8-ol (**5**);
4-(1-Benzylpiperidin-4-yl)-2-(((8-hydroxyquinolin-5-yl)methyl)(prop-2-yny)amino)butanenitrile (**6**);
5-(((3-(1-Benzylpiperidin-4-yl)propyl)(prop-2-ynyl)amino)methyl)quinolin-8-ol (**7**).

11. A pharmaceutical composition comprising a therapeutically effective amount of a compound of formula (I) as defined in any of claims 1-10 or a pharmaceutically acceptable salt thereof together with the appropriate amounts of pharmaceutically acceptable excipients, carriers, or mixtures thereof.

12. A compound of formula (I) as defined in any of claims 1-10 for use as a medicament.

13. A compound of formula (I) as defined in any of claims 1-10 for use in the treatment of a neurodegenerative disorder, disease and/or condition in a mammal, including a human.

14. The compound according to claim 13 wherein the neurodegenerative disorder, disease and/or condition is selected from Alzheimer's disease and Parkinson disease.

15. A process for the preparation of a compound of formula (I) as defined in any of claims 1-10, which comprises reductive amination of a compound of formula (IX) with propargylamine, followed by N-alkylation with 5-chloromethyl-quinolin-8-yl substituted compound of formula (X) 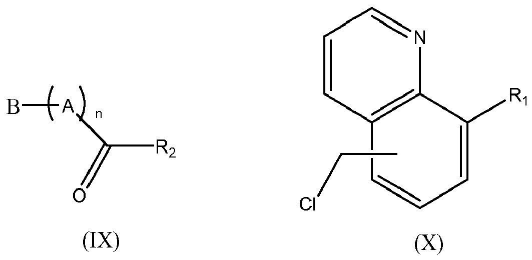 or alternatively, reacting the 5-(chloromethyl)quinolin-8-yl derivative of formula (X) with propargylamine to give the 5-((prop-2-yn-1-ylamino)methyl)quinolin-8-yl derivative of formula (XI), followed by reductive amination with aldehydes of formula (IX) 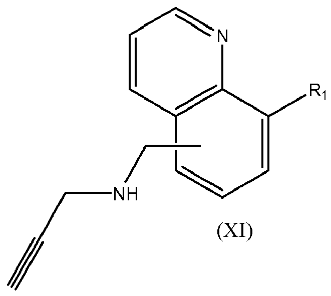 or alternatively, compounds reacting compounds of formula (XII) with propargylamine to provide intermediates of type (XIII), followed by reaction with chlorides of general structure (XIV).
